# EUROPÄISCHE PATENTSCHRIFT

(11) **EP 0 485 045 B1**
(45) Veröffentlichungstag und Bekanntmachung des Hinweises auf die Patenterteilung: **30.12.1998**
(21) Anmeldenummer: 91250305.9
(22) Anmeldetag: 07.11.1991
(51) Int. Cl.: C07D 257/02, A61K 49/00, A61K 31/395

(54) **Mono-N-substituierte 1,4,7,10-Tetraazacyclododecan-Derivate, Verfahren zu ihrer Herstellung und diese enthaltende pharmazeutische Mittel**
Mono-N-substituted 1,4,7,10-Tetraazacyclododecan-derivatives, process for their preparation and pharmaceutical agent containing them
Dérivés du 1,4,7,10-Tétraazacyclododécane-mono-N-substitués, leur procédé de préparation et produits pharmaceutiques les contenant

(30) Priorität: 08.11.1990 DE 4035760
(43) Veröffentlichungstag der Anmeldung: 13.05.1992
(73) Patentinhaber: SCHERING AKTIENGESELLSCHAFT, 13342 Berlin (DE)
(72) Erfinder: Platzek, Johannes, Dr., W-1000 Berlin 33 (DE); Gries, Heinz, Dr., W-1000 Berlin 31 (DE); Weinmann, Hans-Joachim, Dr., W-1000 Berlin 38 (DE); Press, Wolf-Rüdiger, W-1000 Berlin 42 (DE); Vogler, Hubert, Dr., W-1000 Berlin 12 (DE)

(56) Entgegenhaltungen:
- EP-A- 0 255 471
- EP-A- 0 287 465
- EP-A- 0 292 689
- EP-A- 0 299 795
- EP-A- 0 325 762
- EP-A- 0 434 345
- EP-A- 0 434 346
- EP-A- 0 448 191
- US-A- 4 889 931

## Beschreibung

Die Erfindung betrifft den in den Patentansprüchen gekennzeichneten Gegenstand, das heißt neue mono-N-substituierte 1,4,7,10-Tetraazacyclododecan-Derivate, deren Komplexe und Komplexsalze, diese Verbindungen enthaltende Mittel, ihre Verwendung in der Diagnostik sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erste Vertreter dieser Substanzklasse haben sich das in dem europäischen Patent mit der Publikationsnummer 71564 beschriebene Dimegluminsalz des Gd DTPA (Gadolinium-III-Komplex der Diethylentriaminpentaessigsäure) und das in der französischen Patentschrift 2 539 996 beschriebene Megluminsalz der GD DOTA (Gadolinium-III-Komplex des 1,4,7,10-Tetracarboxymethyl-1,4,7,10-Tetraazacyclododecans) als Kontrastmittel für die Kernspintomographie sehr gut bewährt. Sie sind unter den Namen Magnevist^{(R)} und Dotarem^{(R)} registriert worden.

Ein wesentlicher Grund für ihre gute Anwendbarkeit in der Klinik liegt in der hohen Wirksamkeit bei der Kernspintomographie, insbesondere bei vielen Hirntumoren. Sie können deshalb mit 0,1 mmol/kg Körpergewicht sehr viel niedriger dosiert werden als beispielsweise Röntgenkontrastmittel in vielen Röntgenuntersuchungen.

Nun besteht aber der Wunsch, Chelate auch höher dosiert einzusetzen. Das ist besonders zum Nachweis bestimmter Erkrankungen außerhalb des Zentralnervensystems mit Hilfe der Kernspintomographie (NMR-Diagnostik), ganz besonders aber bei der Verwendung von Chelaten als Röntgenkontrastmittel, der Fall.

Um dabei die Volumenbelastung des Körpers möglichst gering zu halten, ist es notwendig, hochkonzentrierte Chelatlösungen zu verwenden. Die bisher bekannten Chelate sind vor allem wegen ihrer zu hohen Osmolalität dafür wenig geeignet.

Es besteht daher ein Bedarf an Chelaten, die eine geringere Osmolalität als die vorbekannten Chelate aufweisen. Gleichzeitig müssen jedoch die Voraussetzungen für die Anwendung dieser Verbindungen am Menschen bezüglich des Abstands zwischen der wirksamen und der im Tierversuch toxischen Dosis (die therapeutische Breite), der Organspezifität, der Stabilität, der kontrastverstärkenden Wirkung, der Verträglichkeit sowie der Löslichkeit der Komplexverbindungen erfüllt sein.

Der Erfindung liegt somit die Aufgabe zugrunde, diese Verbindungen und Mittel zur Verfügung zu stellen, sowie ein möglichst einfaches Verfahren zu ihrer Herstellung zu schaffen. Diese Aufgabe wird durch die vorliegende Erfindung erfüllt.

Die erfindungsgemäßen Komplexverbindungen und die aus ihnen bereiteten Lösungen erfüllen die genannten Anforderungen in überraschender Weise. Sie besitzen eine verringerte Osmolalität sowie eine günstigere therapeutische Breite und/oder Stabilität und Lagerfähigkeit der chemischen Bestandteile der Lösung und/oder Organspezifität und/oder Verträglichkeit (z.B. geringere kardiovaskuläre oder allergieartige Nebenwirkungen) sowie vor allem eine weit bessere kontrastverstärkende Wirkung als die bisher gebräuchlichen Diagnostika.

Als Beleg für die oben genannten überraschenden und hervorragenden Eigenschaften der erfindungsgemäßen Verbindungen sei auf die Übersicht am Ende des experimentellen Teils hingewiesen, in der die als Maß für die kontrastverstärkende Wirkung von NMR-Kontrastmitteln gebräuchlichen Relaxivitäten 1/T₁ (im Plasma) beispielhaft ausgewählter erfindungsgemäßer Komplexe verglichen werden. Die Tabelle zeigt eine deutliche Überlegenheit dieser Verbindungen gegenüber denen als Stand der Technik anzusehenden NMR-Diagnostika Magnevist^{(R)} und Dotarem^{(R)} an.

Überraschenderweise zeigt ein Großteil der erfindungsgemäßen Komplexe ein anderes pharmakokinetisches Verhalten als Magnevist^{(R)} und Dotarem^{(R)}: Nach intravenöser Injektion verteilen sich Magnevist^{(R)} und Dotarem^{(R)} extracellulär und werden durch glomeruläre Sekretion über die Nieren eliminiert. Eine Passage intakter Zellmembranen und eine extrarenale Ausscheidung werden praktisch nicht beobachtet.

Es wurde nun gefunden, daß ein Großteil der erfindungsgemäßen Verbindungen überraschenderweise sowohl renale Ausscheidung als auch Exkretion mit den Faeces nach parenteraler Verabreichung zeigen. Überraschenderweise ist die Elimination über die Galle jedoch nicht der einzige extrarenale Ausscheidungsweg: Bei NMR-Untersuchungen an Ratten wurde nach intravenöser Gabe der erfindungsgemäßen Verbindungen unerwartet auch eine Kontrastverstärkung des Magen-Darm-Trakts beobachtet, d.h. diese Verbindungen sind sowohl fur die organspezifische NMR-Diagnostik des hepatobiliären Systems als auch des Magens geeignet. Die Nieren sowie implantierte Tumore werden ebenfalls kontrastiert.

Die Ausscheidung (Sekretion) über den Magen hat den Vorteil, daß eine Abgrenzung abdominaler Strukturen (z.B. Pankereas) vom Gastrointestinal-Trakt mit gleichzeitiger Kontrastverstärkung pathologischer Prozesse (Tumoren, Entzündungen) ermöglicht wird. Eine Darstellung des renalen Systems, der Leber und der Gallenblase und Gallenwege sowie der Lymphknoten kann darüber hinaus auch erzielt werden. Neben der verbesserten Darstellung von Ulci und Magenkarzinomen kann auch eine Überprüfung der Magensaftsekretion mit Hilfe bildgebender Verfahren durchgeführt werden.

Überraschenderweise werden einige der erfindungsgemäßen Komplexverbindungen auch nach oraler Applikation resorbiert und anschließend hepatobiliär ausgeschieden, d.h. sie sind auch als orale Leberkontrastmittel geeignet.

Durch die Verwendung der o.g. Verbindungen kann - neben der Diagnostik des hepatobiliären Systems - somit sowohl niereninsuffizienten als auch unter gastrointestinalen Erkrankungen leidenden Patienten (mindestens 10 % der Bevölkerung in den westlichen Industrieländern) geholfen werden. Die meisten dieser Patienten sowie eine große Anzahl von Patienten, bei denen der Verdacht auf eine solche Erkrankung vorliegt, müssen sich diagnostischen Untersuchungen unterziehen. Zur Zeit sind für gastrointestinale Erkrankungen vor allem zwei dafür geeigneter Methoden gebräuchlich: die Endoskopie und die Röntgenstrahl-Diagnostik mit Hilfe von Barium-Kontrastmitteln.

Diese Untersuchungen weisen verschiedene Nachteile auf: sie sind mit dem Risiko der Strahlenbelastung behaftet, trauma-verursachend, mit Unannehmlichkeiten, gelegentlich sogar mit Risiken für den Patienten verbunden und können daher psychologischen Streß hervorrufen. Sie müssen meist wiederholt durchgeführt werden, sind relativ aufwendig durchzuführen, erfordern die aktive Mitarbeit des Patienten (z.B. Einnehmen einer bestimmten Körperhaltung) und sind oft nicht anwendbar bei gebrechlichen und bei Risiko-Patienten.

Die Aufgabe, neue diagnostische Methoden zur Erkennung und Lokalisierung gastrointestinaler Erkrankungen, die diese Nachteile nicht besitzen, zur Verfügung zu stellen, wird daher durch die Verwendung der o.g. Komplexverbindungen und Mittel ebenfalls gelöst.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere auch im Falle der Verwendung relativ toxischer Metallionen auch bei hoher Dosierung keine schädlichen Wirkungen beobachtet werden.

Die praktische Anwendung der neuen Komplexe wird auch durch deren günstige chemische Stabilität erleichtert.

Ein weiterer wesentlicher Vorteil der beschriebenen Komplexe und Komplexbildner ist deren außerordentliche chemische Vielseitigkeit. Neben dem Zentralatom lassen sich die Eigenschaften durch die Wahl vielfältiger Substituenten am Makrocyclus und/oder der Salzbildner den Anforderungen an Wirksamkeit, Pharmakokinetik. Verträglichkeit, Löslichkeit, Handhabbarkeit usw. anpassen. So kann z.B. eine in der Diagnostik und Therapie sehr erwünschte Spezifität der Verbindungen für Strukturen im Organismus, für bestimmte biochemische Substanzen, für Stoffwechselvorgänge, für Zustände der Gewebe oder Körperflüssigkeiten erzielt werden.

Die erfindungsgemäßen makrocyclischen Verbindungen werden durch die allgemeine Formel I gekennzeichnet: 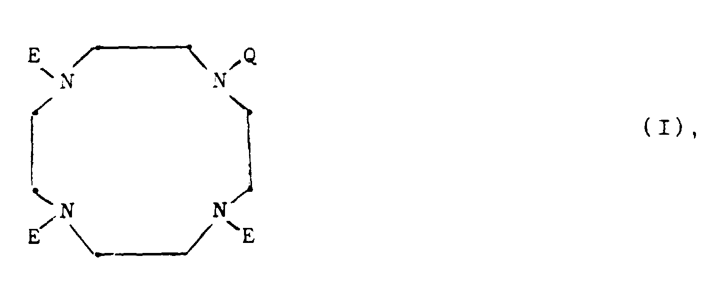 worin
- E: für einen Rest -CH(R¹)-CO₂Y mit Y in der Bedeutung eines Wasserstoffatoms und/oder eines Metallionenäquivalents eines Elements der Ordnungszahlen 21-29, 31, 32, 37-39, 42-44, 47, 49 oder 57-83 und R¹ in der Bedeutung eines Wasserstoffatoms, einer gegebenenfalls mit 1 bis 6 Hydroxygruppen substituierten verzweigten oder unverzweigten C₁-C₃₀-Alkyl-, C₆-C₁₀-Aryl- oder C₇-C₃₀-Aralkylgruppe,
- Q: für einen Rest -CH(R')-CH(OH)R
mit R in der Bedeutung eines 1 bis 10 Sauerstoffatome enthaltenden und gegebenenfalls durch 1 bis 6 Hydroxy- oder OCOR²-Gruppen mit R² in der Bedeutung eines Wasserstoffatoms oder eines verzweigten oder unverzweigten C₁-C₃₀-Alkyl-, C₆-C₁₀-Aryl- oder C₇-C₃₀-Aralkylrestes
substituierten verzweigten oder unverzweigten C₁-C₃₀-Alkyl- oder C₇-C₃₀-Aralkylrestes,
der gegebenenfalls 1 bis 2 -OCO-Gruppen, 1 bis 2 Cyclohexan- und/oder Cyclohexylenreste (die ihrerseits durch 1 bis 3 (CH₂)ₖCOOR²-Gruppen mit k in der Bedeutung der Ziffern 0 bis 10 substituiert sein können), gegebenenfalls 1 bis 5 C₁-C₇-Alkoxy-, C₆-C₁₀-Aryloxy- oder C₇-C₁₀-Aralkoxygruppen, gegebenenfalls einen Rest -NR²-COR³ oder -CONR²R³, wobei R³ die für R² angegebene Bedeutung hat, und/oder 1 bis 2 CO₂R² -Reste enthält und deren gegebenenfalls in der Kette enthaltenen Arylreste durch 1 bis 3, gegebenenfalls 1 bis 5 Sauerstoffatome, 1 bis 3 Hydroxyreste, 1 bis 5 C₁-C₇-Alkoxygruppen und/oder 1 bis 3 (CH₂)ₖCOOR²-Gruppen enthaltende verzweigte oder unverzweigte C₁-C₃₀-Alkyl-, C₆-C₁₀-Aryl- oder C₇-C₃₀-Aralkylreste, 1 bis 3 F-, Cl-, Br-, OH-, NO₂-, NH₂-, NCS-, CO₂R², NHCOCH₂Cl-, NHCOCH₂Br-, NHCOCH₂R²-Reste substituiert sein können, und
- R': in der Bedeutung eines Wasserstoffatoms oder von R, wobei, wenn R' für ein Wasserstoffatom steht, im Falle einer 2-Oxa-C₁-C₃₀-Alkylkette diese - zusätzlich zu einer gegebenenfalls vorhandenen endständigen Methoxy- oder Ethoxygruppe - durch mindestens eine der oben angegebenen Reste substituiert sein muß,
oder für ein über eine Bis(β-hydroxy)-alkylenkette 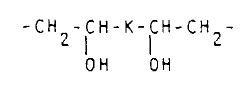 gebundenes zweites Tetraazacyclododecan-Molekül mit K in der Bedeutung einer 1 bis 6 Sauerstoffatome, gegebenenfalls 1 bis 2 Benzyloxy-, Phenylen-, Phenylenoxy- und/oder Phenylendioxygruppen enthaltenen, gegebenenfalls durch 1 bis 6 Hydroxy-, 1 bis 6 Hydroxy-C₁-C₆-alkyl- und/oder 1 bis 8 C₁-C₇-Alkoxy- oder Aralkoxygruppen substituierten C₁-C₁₆-Alkylenkette
stehen sowie deren Salze mit anorganischen und/oder organischen Basen, Aminosäuren oder Aminosäureamiden.

Verbindungen der allgemeinen Formel I mit Y in der Bedeutung von Wasserstoff werden als Komplexbildner und mit mindestens zwei der Substituenten Y in der Bedeutung eines Metallionenäquivalents als Metallkomplexe bezeichnet.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium, Silber, Gold, Rhenium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Der für R¹ und R² gegebenenfalls stehende bzw. in R als Substituent gegebenenfalls enthaltenen Arylrestes gegebenenfalls enthaltene Alkyl-, Aryl- und/oder Aralkylrest enthält 1 bis 30 bzw. C₆-C₁₀, bzw. C₇-C₃₀ C-Atome und kann verzweigt, unverzweigt, cyclisch, gesättigt oder ungesättigt sein, der im Falle von R¹ gegebenenfalls 1 bis 6, vorzugsweise 1 bis 3 Hydroxygruppen, im Falle von R gegebenenfalls 1 bis 5 Sauerstoffatome, 1 bis 3 Hydroxyreste, 1 bis 5, vorzugsweise 1 bis 3 C₁-C₇-Alkoxygruppen und/oder 1 bis 3 (CH₂)ₖCOOR²-Gruppen mit k in der Bedeutung der Ziffern 0 bis 10, vorzugsweise 0 bis 5, enthält.

Als bevorzugte Reste seien beispielhaft genannt: -CH₃, -C₂H₅, -i-C₃H₇, -C₈H₁₇, -C₁₅H₃₁, -C₁₆H₃₃, -C₁₇H₃₅, -CH₂OH, -CH₂CH₂OH, -CH(OH)CH₂OH, -C₆H₅, -CH₂C₆H₅, -CH(OH)CH(OH)CH₂OH, -OCH₃, -OC₂H₅, -OCH₂C₆H₅, -OCH₂CH₂OCH₃, -OCH₂CH₂OCH₂CH₂OH, -(CH₂)₅OH, -CH₂CO₂H, -(CH₂)₂CO₂H, -OCH₂CO₂H, -O(CH₂)₅CO₂H, -CH₂CO₂C₂H₅, -CH₂CO₂iC₃H₇, -CH₂-O-CH(CO₂H)-CH₂CO₂H, -O-CH₂-CH(OCH₃)-CH₂-OCH₃.

R¹ und R' stehen bevorzugt jeweils für ein Wasserstoffatom.

R steht für einen verzweigten, unverzweigten, gesättigten oder ungesättigten C₁-C₃₀-, vorzugsweise für einen C₁-C₂₀-Alkyl- oder C₇-C₃₀-Aralkylrest, der durch 1 bis 10, vorzugsweise 1 bis 5 Sauerstoffatome unterbrochen ist und gegebenenfalls durch 1 bis 6, vorzugsweise 1 bis 3 Hydroxy- oder OCOR²-Gruppen substituiert ist. Er kann 1 bis 2 Cyclohexan- und/oder Cyclohexylenreste, die ihrerseits durch 1 bis 3, vorzugsweise 1 (CH₂)ₖCOOR²- Gruppen substituiert sein können, enthalten.

Weiterhin kann der für R stehende C₁-C₃₀-Rest durch 1 bis 5, vorzugsweise 1 bis 3 C₁-C₇-Alkoxy-, C₆-C₁₀-Aryloxy- oder C₇-C₁₀-Aralkoxygruppen (wie z.B. OCH₃, OC₂H₅, OCH₂C₆H₅, OC₆H₅), einen Rest -NR²COR³ (wie z.B. NHCOC₆H₅, NCH₃COCH₃, NHCOC₂H₅, NCH₃COCH₂C₆H₅), einen Rest -CONR²R³ (wie z.B. CONHCH₃, CONHC₆H₅, CONCH₃C₆H₅, CONH₂, CONHC₃H₇) und/oder 1 bis 2 -CO₂R²-Reste (wie z.B. CO₂CH₃, CO₂C₂H₅, CO₂ⁱPr, CO₂^{t}But, CO₂CH₂C₆H₅) substituiert sein. Die in dieser Kette gegebenenfalls enthaltenen 1 bis 3, vorzugsweise 1 Arylrest(e) können durch 1 bis 3, vorzugsweise 1 bis 2 oben näher beschriebenen C₁-C₁₀-Alkyl-, Aryl-und/oder Aralkylketten und/oder durch 1 bis 3, vorzugsweise 1 bis 2 F-, Cl-, Br-, OH-, NO₂-, NH₂-, NCS-, CO₂R²-, NHCOCH₂Cl-, NHCOCH₂Br-, NHCOCH₂R²-, CON₃-Reste substituiert sein.

Als bevorzugte Reste R seien beispielhaft genannt:
-CH₂-O-C₆H₅, -CH₂-O-CH₂C₆H₅, -CH₂-O-C₁₀H₂₀-OH -CH₂-O-C₁₀H₂₀-COOH, -CH₂-O-C₅H₁₀-COOH, -CH₂CH₂-(C₇H₁₄)-OCH₃, -CH₂-CH₂-CH₂-CH₂-O-CH₂-COOH, -CH₂-O-C₆H₁₀-CO₂H, -CH₂-O-C₆H₁₀-OH, -C₆H₁₂-O-CH₂-COOH, -CH₂-O-C₅H₁₀NHCOC₆H₅, -CH₂-O-C₂H₅NHCOC₆H₅, -CH₂-O-C₂H₅CONH-C₃H₇, -CH₂-O-CH₂CH₂-O-CH₂CH₂-O-C₅H₁₁, -CH₂-O-CH₂-CH(OH)CH₂OH, CH₂-O-C₆H₄NCS, -CH₂-O-C₆H₄OCH₃, -CH₂-O-CH₂CH₂CH₂C₆H₄OCH₃, -CH₂-O-C₆H₄-Cl, -CH₂O-C₆H₄-C₅H₁₀-OH, -CH₂-O-C₆H₃(CH₃)₂, -CH₂-O-C₆H₄(CH₂)₂-COOH, -CH₂-O-C₆H₄-OCH₂COOH, -CH₂-O-C₆H₄COOH, -CH₂O-CH₂-C₆H₄COOH, -CH₂O-C₆H₄CH₂COOH, -CH₂O-C₆H₄CH₂COOC₂H₅, -(CH₂)₉-O-CH₂-C₆H₅, -CH₂-OCO-C₁₅H₃₁, -(CH₂)₉-OCH₃, -CH₂-O-C₉H₁₈-O-CH₂C₆H₅, -CH-O-C₉H₁₈OH, -C₈H₁₆-(CH-O-CH₂-C₆H₅)-C₂H₅, -C₉H₁₈-O-C₆H₄-O-CH₂-C₆H₅, -C₉H₁₈-O-C₆H₄-OH, -(CH₂)₉-O-CH₂-C₆H₄-CH₂COOH, -(CH₂)₉-O-C₁₀H₂₀-OH, -(CH₂)₉-O-C₅H₁₀-COOH, -(CH₂)₉-OCOCH₃, -(CH₂)₉-O-COC₄H₉, -(CH₂)₉-OCOC₂H₄COOH, -(CH₂)₉-OCOC₆H₄COOH, -(CH₂)₉-OCO₆H₁₀COOH.

Zu den erfindungsgemäßen Verbindungen gehören auch Dimere, d.h. Verbindungen der allgemeinen Formel I, worin Q für ein über eine Bis(ß-hydroxy)-alkylenkette 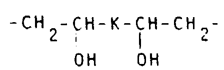 gebundenes zweites Tetraazacyclododecan-Molekül steht. Die Anzahl der in der C₁-C₁₆-, bevorzugt C₁-C₁₀-Alkylenkette K enthaltenen Sauerstoffatome ist vorzugsweise 1 bis 3. Sie enthält gegebenenfalls 1 bis 6 Hydroxy- und/oder 1 bis 6 Hydroxy-C₁-C₆-alkyl- und/oder 1 bis 8, vorzugsweise 1 bis 2 C₁-C₇-Alkoxy-, Aryloxy- und/oder Aralkoxygruppen.

Als bevorzugte Alkylenkette K seien beispielhaft genannt:
-CH₂-O-CH₂-, -CH₂O-CH₂CH₂-O-CH₂-, -CH₂-O-C₃H₆-O-CH₂-, -CH₂-O-C₄H₈-O-CH₂-, -CH₂O-CH(CH₂OH)-CH₂-CH(CH₂OH)-O-CH₂-, -CH₂O-CH₂-CH(OH)-CH₂-O-CH₂-, -CH₂-O-CH₂-CH(OCH₃)CH₂-O-CH₂-, -CH₂-OCH₂-C(CH₂OH)₂-CH₂-O-CH₂-, -CH₂-O-C₆H₄-O-CH₂-, -CH₂-O-CH₂-C₆H₄-CH₂-O-CH₂-, -CH₂-O-CH₂-CH(OH)-CH(OH)-CH₂-O-CH₂-, -CH₂-O-CH₂-CH(CH₂OH)-CH₂-O-CH₂-, -CH₂-O-C₆H₄-O-C₆H₄-O-CH₂-.

Die restlichen aciden Wasserstoffatome, das heißt diejenigen, die nicht durch das Zentralion substituiert worden sind, können gegebenenfalls ganz oder teilweise durch Kationen anorganischer und/oder organischer Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren wie z.B. Lysinmethylamid, Glycinethylamid und Serinmethylamid.

Die Herstellung der erfindungsgemäßen Tetraazacyclododecan-Verbindungen der allgemeinen Formel I erfolgt dadurch, daß man in an sich bekannter Weise Verbindungen der allgemeinen Formel II 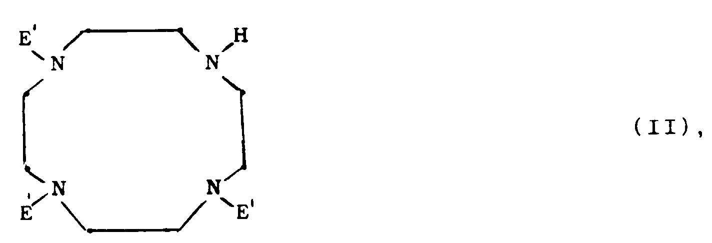 worin
- E': den für E angegebenen Rest, in dem darin gegebenenfalls vorhandene Hydroxygruppen gegebenenfalls geschützt sind,
bedeutet,
mit einem Edukt der allgemeinen Formel III bzw. IV 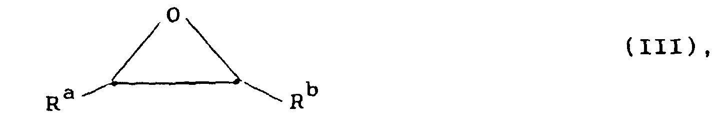 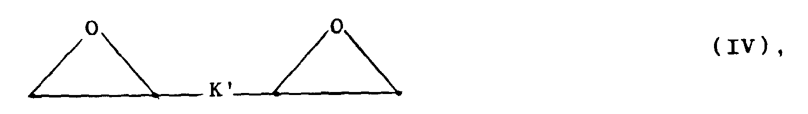 worin
- K': den für K angegebenen Rest, in dem darin gegebenenfalls vorhandene Hydroxygruppen gegebenenfalls geschützt sind, bedeutet
- R^{a} und R^{b}: die für R' und R angegebene Bedeutung, jedoch keine Gruppen NH₂, NCS, NHCOCH₂Cl oder NHCOCH₂Br enthalten und gegebenenfalls vorhandene Carboxylgruppen gewünschtenfalls in geschützter Form vorliegen, haben,
in Wasser oder in mit Wasser mischbaren Lösungsmitteln wie z.B. Acetonitril, DMF, DMA, Ethanol, Methanol, Dioxan, THF, DMSO, DME oder deren Gemischen bei Temperaturen von 0 °C bis 170 °C, vorzugsweise 20 bis 100 °C, innerhalb von 12 bis 48 Stunden, vorzugsweise 12 bis 24 Stunden, unter Zusatz von anorganischen und/oder organischen Basen, wie z.B. Kalium-, Natrium-, Lithium-, Calcium-, Barium-, Magnesiumhydroxid, Natrium-, Kaliumcarbonat, Triethyl-, Tripropyl-, Tributylamin, Pyridin, DMAP, Reillex^{(R)}, Triton B^{(R)} umsetzt, gegebenenfalls noch vorhandene Schutzgruppen spaltet, die so erhaltenen Komplexbildner (Y steht für Wasserstoffatome) mit mindestens einem Metalloxid oder Metallsalz eines Elements der Ordnungszahlen 21-29, 31, 32, 37-39, 42-44, 47, 49 oder 57-83 umsetzt und anschließend - falls gewünscht - vorhandene acide Wasserstoffatome durch Kationen anorganischer und/oder organischer Basen, Aminosäuren oder Aminosäureamide substituiert, wobei - falls die gewünschten Endprodukte die Gruppen NH₂, NCS, NHCOCH₂Cl, NHCOCH₂Br bzw. CON₃ enthalten sollen - man von Edukten der allgemeinen Formel III ausgeht, die anstelle dieser Gruppen NO₂- bzw. CO₂R²-Gruppen enthalten, anschließend die letztgenannten Gruppen in die oben genannten gewünschten funktionellen Gruppen umwandelt, wobei diese Umwandlung vor oder nach der Komplexierung der Komplexbildner und anschließender Substitution gegebenenfalls noch vorhandener acider Wasserstoffatome mit den jeweils gewünschten Metallionen erfolgen kann.

Als Hydroxyschutzgruppen kommen alle diejenigen infrage, die sich leicht einführen und später unter Rückbildung der letztlich gewünschten freien Hydroxygruppe auch wieder leicht abspalten lassen. Bevorzugte Schutzgruppen sind Ethergruppen wie zum Beispiel die Benzyl-, 4-Methoxybenzyl-, 4-Nitrobenzyl-, Trityl-, Di- und Triphenylmethyl-, Trimethylsilyl-, Dimethyl-t-butylsilyl-, Diphenyl-t-butylsilylgruppe. Bevorzugt sind die Hydroxygruppen jedoch in Form von Ketalen mit zum Beispiel Aceton, Acetaldehyd, Cyclohexanon oder Benzaldehyd geschützt.

Die Abspaltung der Hydroxyschutzgruppen erfolgt in an sich bekannter Weise, zum Beispiel im Falle eines Benzylethers durch reduktive Spaltung mit Lithium/Ammoniak oder durch hydrogenolytische Spaltung in Gegenwart von zum Beispiel Palladium-Kohle und im Falle einer Ether- oder Ketalspaltung durch Säurebehandlung mit Hilfe von zum Beispiel Kationenaustauschern, Trifluoressigsäure oder Mineralsäuren [siehe z.B. T.W. Greene "Protective Groups in Organic Synthesis", John Wiley and Sons (1991)].

Als Säureschutzgruppen kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Bei der Umsetzung der z.B. aus den Europäischen Patentanmeldungen EP 255 471 und EP 287 465 bekannten trisubstituierten Tetraazacyclododecan-Derivate II mit den Epoxiden III arbeitet man mit einem 1,05 bis 5fachen, bevorzugt 1,05 bis 3fachen Uberschuß an III.

Im Unterschied dazu wird bei der Synthese von dimeren Makrocyclen mit 0,1 bis 0,49 Aquivalenten IV umgesetzt.

Die so erhaltenen Komplexbildner werden nach Reinigung an Kieselgel-Säulen, Reversed-phase-Säulen (RP-18) oder an Ionenaustauscher-Säulen (z.B. IRA 67, IR 120, AMB 252) isoliert.

Die gegebenenfalls im Endprodukt gewünschten funktionellen Gruppen NH₂, NHCOCH₂Cl, NHCOCH₂Br und NCS werden nach der Alkylierung mit einem eine NO₂-Gruppe enthaltenen Epoxid III (entweder vor oder nach der gegebenenfalls gewünschten Metalleinführung in den Makrocyclus) nach literaturbekannten Methoden (Europäische Patentanmeldung Publikations-Nr.: 292 689; US-Patent 4,678,667; Bioconjugate Chem. 1990, 1, 59; J. Med. Chem. 1989, 32, 236) generiert.

Die als Edukte benötigten Epoxide III und IV sind bekannt oder können analog literaturbekannten Methoden hergestellt werden, z.B. durch Umsetzung von Alkoholen mit Epichlorhydrin (Synthesis 1983, 117; Houben-Weyl, Methoden der Organischen Chemie, Band 613, Georg-Thieme-Verlag Stuttgart, 1965) und Epoxidierung von substituierten Allylalkoholen oder ungesättigten Ethern [J. Org. Chem. 35, 215 (1970), Houben-Weyl, Georg-Thieme-Verlag Stuttgart 1965; Tetrahedron Lett. 1965, 849].

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat des Elements der Ordnungszahlen 21-29, 31, 32, 37-39, 42-44, 47, 49, 57-83) in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Einführung der gewünschten Metallionen kann dabei sowohl vor als auch nach der Abspaltung der Hydroxyschutzgruppen erfolgen.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy-Gruppen mit Dimethylsulfat).

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Olen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 0,1 µMol - 3 Mol/l des Komplexsalzes und werden in der Regel in Mengen von 0,1 µMol - 5 mMol/kg dosiert. Sie sind zur enteralen und parenteralen Applikation bestimmt. Die erfindungsgemäßen Komplexverbindungen kommen zur Anwendung:
1.) für die NMR- und Röntgen-Diagnostik in Form ihrer Komplexe mit den Ionen der Elemente mit den Ordnungszahlen 21 - 29, 42, 44 und 57-83;
2.) für die Radiodiagnostik und Radiotherapie in Form ihrer Komplexe mit den Radioisotopen der Elemente mit den Ordnungszahlen 21, 26, 27, 29, 31, 32, 37-39, 43, 47, 49, 62-64, 67, 70, 71, 75, 77, 79 und 83.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der Methode, z.B. Brachytherapie.

Die erfindungsgemäßen therapeutischen Mittel werden parenteral appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

### a) 1,1'-(2,9-Dihydroxy-4,7-dioxa-1,10-decyl)-bis-[(4,7,10-tris-carboxymethyl)-1,4,7,10-tetraazacyclododecan]

17,9 g (51,67 mmol) 1,4,7-Triscarboxymethyl-1,4,7,10-tetraazacyclododecan (DO3A) werden in 80 ml Wasser gelöst und der pH mit 5 normaler Natronlauge auf 13 eingestellt. Dann wird innerhalb einer Stunde eine Lösung aus 3 g (17,22 mmol) 1,2-9,10-Diepoxy-4,7-dioxadecan in 30 ml Dioxan zugetropft und 24 Stunden bei 50°C gerührt. Die Lösung wird mit 10%iger Salzsäure auf pH 2 gestellt und im Vakuum eingedampft. Der Rückstand wird in etwas Wasser gelöst und an einer lonenaustauschersäule (Reillex® = Poly-(4-vinylpyridin)/man eluiert mit Wasser) gereinigt. Die Hauptfraktionen werden im Vakuum eingedampft und der Rückstand durch Chromatographie an RP-18 (LiChroPrep®/Laufmittel: Gradient aus Tetrahydrofuran/Methanol/Wasser) endgereinigt. Nach Eindampfen der Hauptfraktionen erhält man 5,82 g (39 % d. Th.) eines amorphen Feststoffes.

| Analyse (bezogen auf wasserfreie Substanz): | | | |
|---|---|---|---|
| C 49,87 | H 7,67 | N 12,92 | ber. |
| C 49,80 | H 7,81 | N 12,88 | |

### b) Bis-Gadolinium-Komplex von 1,1'-(2,9-Dihydroxy-4,7-dioxa-1,10-decyl)-bis[(4,7,10-triscarboxymethyl)-1,4,7,10-tetraazacyclododecan

5 g (5,77 mmol) der Titelverbindung aus Beispiel 1a werden in 40 ml entionisiertem Wasser gelöst und 2,09 g (5,77 mmol) Gadoliniumoxid zugesetzt. Man erwärmt 3 Stunden auf 90°C. Die abgekühfte Lösung wird mit je 2 ml saurem Ionenaustauscher (IR-120) und 2 ml basichem Austauscher (IRA-410) eine Stunde bei Raumtemperatur gerührt. Man filtriert vom Austauscher ab und kocht das Filtrat mit Aktivkohle kurz auf. Nach Filtration und Gefriertrocknung erhält man 6,58g (97 % d. Th.) eines amorphen Pulvers.

| Analyse (bezogen auf wasserfreie Substanz): | | | | |
|---|---|---|---|---|
| C 36,79 | H 5,14 | N 9,53 | Gd 26,76 | ber. |
| C 36,71 | H 5,21 | N 9,46 | Gd 26,71 | |

### Beispiel 2

### a) 1,1'-(2,6-Dihydroxy-4-oxa-1,7-heptyl)-bis-[(4,7,10-triscarboxymethyl)-1,4,7,10-tetraazacyclododecan]

Analog zu Beispiel 1a wurde 1,2-6,7-Diepoxy-4-oxa-heptan anstelle von 1,2-9,10-Diepoxy-4,7-dioxadecan eingesetzt.
Ausbeute: 32 % d. Th. eines glasigen Feststoffes

| Analyse (bezogen auf wassertreie Substanz): | | | |
|---|---|---|---|
| C 49,63 | H 7,59 | N 13,62 | ber. |
| C 49,58 | H 7,65 | N 13,54 | |

### b) Bis-Gadolinium-Komplex von 1,1'-(2,6-Dihydroxy-4-oxa-1,7-heptyl)-bis-[(4,7,10-triscarboxymethyl)-1,4,7,10-tetraazacyclododecan]

Analog zu Beispiel 1b wurde die Titelverbindung aus Beispiel 2a anstelle von der Titelverbindung 1a zur Komplexierung eingesetzt.
Ausbeute: 96 % d. Th. eines farblosen, amorphen Pulvers

| Analyse (bezogen auf wasserfreie Substanz): | | | | |
|---|---|---|---|---|
| C 36,10 | H 4,99 | N 9,90 | Gd 27,80 | ber. |
| C 36,02 | H 5,07 | N 9,82 | Gd 27,73 | |

### c) Bis-Ytterbium-Komplex von 1,1'-(2,6-Dihydroxy-4-oxa-1,7-heptyl)-bis-[(4,7,10-triscarboxymethyl)-1,4,7,10-tetraazacyclododecan]

Analog zu Beispiel 1b wurde Ytterbiumoxid anstelle von Gadoliniumoxid eingesetzt.
Ausbeute: 97 % d. Th. eines glasigen Feststoffes

| Analyse (bezogen auf wasserfreie Substanz): | | | | |
|---|---|---|---|---|
| C 35,11 | H 4,85 | N 9,64 | Yb 29,76 | ber. |
| C 35,03 | H 4,92 | N 9,57 | Yb 29,68 | |

### d) Bis-Dysprosium-Komplex von 1,1'-(2,6-Dihydroxy-4-oxa-1,7-heptyl)-bis-[(4,7,10-triscarboxymethyl)-1,4,7,10-tetraazacyclododecan]

Analog zu Beispiel 1b wurde Dysprosiumoxid anstelle von Gadoliniumoxid eingesetzt.
Ausbeute: 98 % d. Th. eines amorphen Pulvers

| Analyse (bezogen auf wasserfreie Substanz): | | | | |
|---|---|---|---|---|
| C 35,76 | H 4,94 | N 9,81 | Dy 28,46 | ber. |
| C 35,66 | H 5,03 | N 9,74 | Dy 28,40 | |

### e) Bis-Wismut-Komplex von 1,1'-(2,6-Dihydroxy-4-oxa-1,7-heptyl)-bis-[(4,7,10-triscarboxymethyl)-1,4,7,10-tetraazacyclododecan]

Analog zu Beispiel 1b wurde Wismutoxid anstelle von Gadoliniumoxid eingesetzt.
Ausbeute: 95 % d. Th. eines farblosen, amorphen Pulvers

| Analyse (bezogen auf wasserfreie Substanz): | | | | |
|---|---|---|---|---|
| C 33,07 | H 4,57 | N 9,07 | Bi 33,85 | ber. |
| C 32,98 | H 4,63 | N 9,00 | Bi 33,78 | |

### Beispiel 3

### a) 1,1'-(2,11-Dihydroxy-4,9-dioxa-dodecyl)-bis-[(4,7,10-triscarboxymethyl)-1,4,7,10-tetraazacyclododecan]

Analog zu Beispiel 1a wurde 1,2-11,12-Diepoxy-4,9-dioxa-4-dodecan anstelle von 1,2-9,10-Diepoxy-4,7-dioxadecan eingesetzt.
Ausbeute: 37 % d. Th.eines farblosen Feststoffes

| Analyse (bezogen auf wasserfreie Substanz): | | | |
|---|---|---|---|
| C 51,00 | H 7,88 | N 12,52 | ber. |
| C 49,93 | H 7,96 | N 12,47 | |

### b) Bis-Gadolinium-Komplex von 1,1'-(2,11-Dihydroxy-4,9-dioxa-dodecyl)-bis-[(4,7,10-triscarboxymethyl)-1,4,7,10-tetraazacyclododecan]

Analog zu Beispiel 1b wurde die Titelverbindung aus Beispiel 3a anstelle von der Titelverbindung 1a zur Komplexierung eingesetzt.
Ausbeute: 98 % d. Th. eines amorphen Pulvers

| Analyse (bezogen auf wasserfreie Substanz): | | | | |
|---|---|---|---|---|
| C 37,92 | H 5,36 | N 9,31 | Gd 26,13 | ber. |
| C 37,84 | H 5,41 | N 9,24 | Gd 26,08 | |

### Beispiel 4

### a) 10-(2-Hydroxy-11-benzyloxy-undecyl)-1,4,7-tris-carboxymethyl-1,4,7,10-tetraazacyclododecan

28,73 g (103,92 mmol) 1,2-Epoxy-11-benzyloxyundecan und 10g (28,86 mmol) 1,4,7-Triscarboxymethyl-1,4,7,10-tetraazacyclododecan (=DO3A) werden in einer Mischung aus 50 ml Dioxan/80 ml Wasser gelöst, und der pH-Wert mit 6N Kalilauge auf pH 10 gebracht. Man rührt 24 Stunden bei 70°C. Man dampft zur Trockene ein, nimmt den Rückstand mit 200 ml Wasser/50 ml Methanol auf und extrahiert 2 mal mit 100 ml tert.-Butyl-methylether. Die wäßrige Lösung wird mit 5N-Salzsäure auf pH 3 gestellt und zur Trockene eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 12,22g (68 % d. Th.) eines stark hygroskopischen, glasigen Feststoffes.

| Analyse (bezogen auf wasserfreie Substanz): | | | |
|---|---|---|---|
| C 61,71 | H 8,74 | N 9,00 | ber. |
| C 61,82 | H 8,91 | N 8,87 | |

### b) Gadolinium-Komplex von 10-(2-Hydroxy-11-benzyloxy-undecyl)-1,4,7-tris-carboxymethyl-1,4,7,10-tetraazacyclododecan

10,77 g (17,3 mmol) der Titelverbindung aus Beispiel 4a werden in 50 ml entionisiertem Wasser gelöst und 3,13 g (8,65 mmol) Gadoliniumoxid zugesetzt. Es wird 3 Stunden auf 90°C erhitzt. Die abgekühlte Lösung wird eine Stunde mit 3 ml saurem Ionenaustauscher (AMB 252c) und 3 ml schwach basischem Austauscher (IRA 67) gerührt. Es wird vom Austauscher abfiltriert und das Filtrat gefriergetrocknet.
Ausbeute: 13,17g (98 % d. Th.) eines farblosen, amorphen Pulvers

| Analyse (bezogen auf wasserfreie Substanz): | | | | |
|---|---|---|---|---|
| C 49,46 | H 6,62 | N 7,21 | Gd 20,24 | ber. |
| C 49,23 | H 6,81 | N 7,15 | Gd 20,13 | |

### c) Eisen-Komplex von 10-(2-Hydroxy-11-benzyloxy-undecyl)-1,4,7-tris-carboxymethyl-1,4,7,10-tetraazacyclododecan

Analog zu Beispiel 4b wurde Eisen(III)oxid anstelle von Gadoliniumoxid eingesetzt.
Ausbeute: 89 % d. Th. eines gelblichen Pulvers

| Analyse (bezogen auf wasserfreie Substanz): | | | | |
|---|---|---|---|---|
| C 56,88 | H 7,61 | N 8,29 | Fe 8,27 | ber. |
| C 56,69 | H 7,52 | N 8,34 | Fe 8,18 | |

### d) Mangan-Komplex von 10-(2-Hydroxy-11-benzyloxy-undecyl)-1,4,7-tris-carboxymethyl-1,4,7,10-tetraazacyclododecan (als Natriumsalz)

Analog zu Beispiel 4b wurde Mangan(2)carbonat anstelle von Gadoliniumoxid eingesetzt. Es wird jedoch nur mit saurem Austauscher (IR-120) nachgerührt. Nach Filtration vom Austauscher wird das Filtrat mit 1N Natronlauge auf pH 7,2 gebracht und anschließend gefriergetrocknet.
Ausbeute: 93 % d. Th. eines glasigen Feststoffes

| Analyse (bezogen auf wasserfreie Substanz): | | | | | |
|---|---|---|---|---|---|
| C 55,09 | H 7,37 | N 8,03 | Mn 7,87 | Na 3,30 | ber. |
| C 55,01 | H 7,44 | N 8,17 | Mn 7,71 | Na 3,17 | |

### Beipiel 5

### a) 1,2-Epoxy-11-methoxy-undecan

9,35 g (51,3 mmol) 1-Methoxy-undec(10)en werden in 100 ml Chloroform gelöst. Bei 0°C setzt man 11,51 g (66,7 mmol) m-Chlorperoxybenzoesäure zu und läßt anschließend auf Raumtemperatur erwärmen. Man rührt 24 Stunden bei Raumtemperatur. Der ausgefallene Niederschlag wird abfiltriert und das Filtrat mehrmals mit konz. Natriumcarbonat-Lösung gewaschen. Die organische Phase wird über Magnesiumsulfat getrocknet und im Vakuum eingedampft. Der Rückstand wird an Kieselgel (Laufmittel: Hexan/Essigester 15:1) chromatographiert.
Ausbeute: 8,76 g (87 % d. Th.) eines farblosen Öls

| Analyse (bezogen auf wasserfreie Substanz): | | |
|---|---|---|
| C 73,41 | H 12,32 | ber. |
| C 73,28 | H 12,41 | |

### b) 10-(2-Hydroxy-11-methoxyundecyl)-1,4,7-tris-carboxymethyl-1,4,7,10-tetraazacyclododecan

Analog zu Beispiel 4a wurde 1,2-Epoxy-11-methoxy-undecan anstelle des in Beispiel 4a benutzten Epoxids eingesetzt.
Ausbeute: 75 % d. Th. eines glasigen Feststoffes

| Analyse (bezogen auf wasserfreie Substanz): | | | |
|---|---|---|---|
| C 57,12 | H 9,22 | N 10,25 | ber. |
| C 57,01 | H 9,34 | N 10,12 | |

### c) Gadolinium-Komplex von 10-(2-Hydroxy-11-methoxyundecyl)-1,4,7-tris-carboxymethyl-1,4,7,10-tetraazacyclododecan

Analog zu Beispiel 4b wurde die Titelverbindung aus Beispiel 5b anstelle der Titelverbindung aus Beispiel 4a zur Komplexierung eingesetzt.
Ausbeute: 97 % d. Th. eines farblosen, amorphen Pulvers

| Analyse (bezogen auf wasserfreie Substanz): | | | | |
|---|---|---|---|---|
| C 44,55 | H 6,76 | N 8,00 | Gd 22,43 | ber. |
| C 44,40 | H 6,83 | N 7,87 | Gd 22,31 | |

### d) Dysprosium-Komplex von 10-(2-Hydroxy-11-methoxyundecyl)-1,4,7-tris-carboxymethyl-1,4,7,10-tetraazacyclododecan

Analog zu Beispiel 5c wurde Dysprosiumoxid anstelle von Gadoliniumoxid eingesetzt.
Ausbeute: 97 % d. Th. eines amorphen Pulvers

| Analyse (bezogen auf wasserfreie Substanz): | | | | |
|---|---|---|---|---|
| C 44,22 | H 6,71 | N 7,93 | Dy 23,01 | ber. |
| C 44,10 | H 6,83 | N 7,85 | Dy 22,87 | |

### Beispiel 6

### a) 1-Hydroxy-9-benzyloxy-nonan

16 g (100 mmol) Nonan-1,9-diol wurden in 150 ml Dimethylformamid gelöst und zu einer Suspension von 2,88 g (1,20 mmol) Natriumhydrid in 100 ml DMF zugetropft. Man rührt 2 Stunden bei 50°C. Man kühlt im Eisbad auf 0°C und tropft innerhalb 3 Stunden 17,1 g (100 mmol) Benzylbromid zu. Dann wird 5 Stunden bei 60°C gerührt. Die Lösung wird auf Raumtemperatur abgekühlt, 1 l Wasser zugegeben und 3 mal mit 150 ml Ether extrahiert. Die vereinigten organischen Phasen werden über Magnesiumsulfat getrocknet und im Vakuum eingedampft. Das zurückbleibende Öl wird an Kieselgel chromatographiert (Laufmittel: Hexan/Aceton=15:1)
Ausbeute: 16,53 g (66 % d. Th.) eines farblosen Öls

| Analyse (bezogen auf wasserfreie Substanz): | | |
|---|---|---|
| C 76,75 | H 10,47 | ber. |
| C 76,63 | H 10,53 | |

### b) 1,2-Epoxy-4-oxa-13-benzyloxy-tridecan

Zu 31 g (123,74 mmol) 1-Hydroxy-9-benzyloxynonan in 400 ml Dimethylformamid gibt man 3,56 g (148,5 mmol) Natriumhydrid und rührt 1 Stunde bei Raumtemperatur (unter Stickstoff). Man gibt 34,35 g (371,22 mmol) Epichlorhydrin zu und erwärmt anschließend 24 Stunden bei 70°C. Es wird im Eisbad auf 0°C abgekühlt und vorsichtig 800 ml Wasser zugegeben. Anschließend wird 2 mal mit je 350 ml Ether extrahiert. Die vereinigten Etherphasen werden 1 mal mit 300 ml Wasser gewaschen, über Magnesiumsulfat getrocknet und im Vakuum eingedampft. Der Rückstand wird an Kieselgel chromatographiert (Laufmittel: Hexan/Essigester = 20:1).
Ausbeute: 26,92 g (71 % d. Th.) eines farblosen Öls

| Analyse (bezogen auf wasserfreie Substanz): | | |
|---|---|---|
| C 74,47 | H 9,87 | ber. |
| C 74,29 | H 9,98 | |

### c) 10-(2-Hydroxy-4-oxa-13-benzyloxy-tridyl)-1,4,7-triscarboxymethyl-1,4,7,10-tetraazacyclododecan

Analog zu Beispiel 4a wurde die Titelverbindung aus Beispiel 6b anstelle des in 4a benutzten Epoxids eingesetzt.
Ausbeute: 68 % d. Th. eines glasigen Feststoffes

| Analyse (bezogen auf wasserfreie Substanz): | | | |
|---|---|---|---|
| C 60,71 | H 8,65 | N 8,58 | ber. |
| C 60,55 | H 8,74 | N 8,47 | |

### d) Gadolinium-Komplex von 10-(2-Hydroxy-4-oxa-13-benzyloxy-tridecyl)-1,4,7-triscarboxymethyl-1,4,7,10-tetraazacyclododecan

Analog zu Beispiel 4b wurde die Titelverbindung aus Beispiel 6c anstelle der Titelverbindung aus Beispiel 4a zur Komplexierung eingesetzt.
Ausbeute: 95 % d. Th. eines glasigen Feststoffes

| Analyse (bezogen auf wasserfreie Substanz): | | | | |
|---|---|---|---|---|
| C 49,11 | H 6,62 | N 6,94 | Gd 19,48 | ber. |
| C 49,02 | H 6,73 | N 6,85 | Gd 19,31 | |

### Beispiel 7

### 10-(2,13-Dihydroxy-4-oxa-tridecyl)-1,4,7-triscarboxymethyl-1,4,7,10-tetraazacyclododecan

15 g (18,59 mmol) der Titelverbindung aus Beispiel 6d werden in einer Mischung aus 100 ml Wasser/100 ml Methanol gelöst und 3 g Palladiumhydroxid (Pearlman-Katalysator) zugegeben. Anschließend wird bei 40°C unter Normaldruck hydriert (nach 2 Stunden vollständige Umsetzung laut HPLC). Man filtriert vom Katalysator ab und dampft das Filtrat im Vakuum zur Trockene ein.
Ausbeute: 13,06 g (98 % d. Th.) eines farblosen amorphen Pulvers

| Analyse (bezogen auf wasserfreie Substanz): | | | | |
|---|---|---|---|---|
| C 43,56 | H 6,61 | N 7,81 | Gd 21,93 | ber. |
| C 43,41 | H 6,79 | N 7,70 | Gd 21,80 | |

### Beispiel 8

### a) 10-(2,6,7-Trihydroxy-4-oxa-heptyl)-1,4,7-tris-carboxymethyl-1,4,7,10-tetraazacyclododecan

Analog zu Beispiel 4a wurde 2,2-Dimethyl-4-(2',3'-epoxy)-propoxy-methyl-1,3-dioxolan anstelle des in Beispiel 4a benutzten Epoxids eingesetzt.
Ausbeute: 71 % d. Th. eines farblosen Feststoffes

| Analyse (bezogen auf wasserfreie Substanz): | | | |
|---|---|---|---|
| C 46,57 | H 7,74 | N 11,33 | ber. |
| C 48,46 | H 7,81 | N 11,24 | |

### b) Gadolinium-Komplex von 10-(2,6,7-Trihydroxy-4-oxa-heptyl)-1,4,7-tris-carboxymethyl-1,4,7,10-tetraazacyclododecan

Analog zu Beispiel 4b wurde die Titelverbindung aus Beispiel 8a anstelle von der Titelverbindung 4a zur Komplexierung eingesetzt.
Ausbeute: 98 % d. Th. eines amorphen Pulvers

| Analyse (bezogen auf wasserfreie Substanz): | | | | |
|---|---|---|---|---|
| C 37,03 | H 5,44 | N 8,64 | Gd 24,24 | ber. |
| C 37,00 | H 5,51 | N 8,57 | Gd 24,18 | |

### c) Europlum-Komplex von 10-(2,6,7-Trihydroxy-4-oxa-heptyl)-1,4,7-tris-carboxymethyl-1,4,7,10-tetraazacyclododecan

Analog zu Beispiel 8b wurde Europiumoxid anstelle von Gadoliniumoxid eingesetst.
Ausbeute: 96 % d. Th. eines amorphen Pulvers

| Analyse (bezogen auf wasserfreie Substanz): | | | | |
|---|---|---|---|---|
| C 37,33 | H 5,48 | N 8,71 | Eu 23,62 | ber. |
| C 37,28 | H 5,53 | N 8,66 | Eu 23,56 | |

### d) Dysprosium-Komplex von 10-(2,6,7-Trihydroxy-4-oxa-heptyl)-1,4,7-tris-carboxymethyl-1,4,7,10-tetraazacyclododecan

Analog zu Beispiel 8b wurde Dysprosiumoxid anstelle von Gadoliniumoxid eingesetzt.
Ausbeute: 96% d. Th. eines amorphen Feststoffes

| Analyse (bezogen auf wasserfreie Substanz): | | | | |
|---|---|---|---|---|
| C 36,73 | H 5,39 | N 8,57 | Dy 24,85 | ber. |
| C 36,67 | H 5,44 | N 8,51 | Dy 24,80 | |

### e) Ytterbium-Komplex von 10-(2,6,7-Trihydroxy-4-oxa-heptyl)-1,4,7-tris-carboxymethyl-1,4,7,10-tetraazacyclododecan

Analog zu Beispiel 8b wurde Ytterbiumoxid anstelle von Gadoliniumoxid eingesetzt.
Ausbeute: 97 % d. Th. eines amorphen Pulvers

| Analyse (bezogen auf wasserfreie Substanz): | | | | |
|---|---|---|---|---|
| C 36,14 | H 5,31 | N 8,43 | Yb 26,04 | ber. |
| C 36,19 | H 5,28 | N 8,38 | Yb 25,96 | |

### Beispiel 9

### a) 2,5,8,13,16,19-hexoxaelcos-10-en

Zu 40 g (332,9 mmol) Diethylenglycolmonomethylether in 500 ml Dimethylformamid gibt man 8,79 g (366,2 mmol) Natriumhydrid und rührt 1 Stunde bei Raumtemperatur. Dann gibt man 23,74 g (111 mmol) cis-1,4-Dibrom-2-buten zu und erwärmt 24 Stunden bei 70°C. Es wird im Eisbad auf 0°C abgekühlt und vorsichtig 1000 ml Wasser zugegeben. Anschließend wird 2 mal mit je 400 ml Ether extrahiert. Die vereinigten Etherphasen werden 1 mal mit 200 ml Wasser gewaschen, über Magnesiumsulfat getrocknet und im Vakuum eingedampft. Der Rückstand wird an Kieselgel (Laufmittel: Hexan/Essigester 20:1) chromatographiert.
Ausbeute: 15,25 g (47 % d. Th. bzgl. Dibrombuten) eines farblosen Öls

| Analyse (bezogen auf wasserfreie Substanz): | | |
|---|---|---|
| C 57,51 | H 9,65 | ber. |
| C 57,58 | H 9,57 | |

### b) 10,11-Epoxy-2,5,8,13,16,19-hexoxaeicosan

15 g (51,3 mmol) der Titelverbindung aus Beispiel 9a werden in 100 ml Chloroform gelöst. Bei 0°C setzt man 11,51 g (66,7 mmol) m-Chlorperoxybenzoesäure zu und läßt anschließend auf Raumtemperatur erwärmen. Man rührt 24 Stunden bei Raumtemperatur. Der ausgefallene Niederschlag wird abfiltriert und das Filtrat mehrmals mit konz. Natriumcarbonat-Lösung gewaschen. Die organische Phase wird über Magnesiumsulfat getrocknet und im Vakuum eingedampft. Der Rückstand wird an Kieselgel (Laufmittel: Hexan/Essigester 15:1) chromatographiert.
Ausbeute: 13,76 (87 % d. Th.) eines faerblosen Öls

| Analyse (bezogen auf wasserfreie Substanz): | | |
|---|---|---|
| C 54,53 | H 9,15 | ber. |
| C 54,47 | H 9,06 | |

### c) 10-[1-(2,5,8-Trioxanonyl)-2-hydroxy-4,7,10-trioxa-undecanyl)]-1,4,7-triscarboxymethyl-1,4,7,10-tetraazacyclododecan

Analog zu Beispiel 4a wurde 10,11-Epoxy-2,5,8,13,16,19-hexoxaeicosan anstelle des in Beispiel 4a benutzten Epoxids eingesetzt.
Ausbeute: 34 % d. Th. eines glasigen Feststoffes

| Analyse (bezogen auf wasserfreie Substanz): | | | |
|---|---|---|---|
| C 51,36 | H 8,31 | N 8,56 | ber. |
| C 51,27 | H 8,40 | N 8,51 | |

### d) Gadolinium-Komplex von 10-[1-(2,5,8-Trioxanonyl)-2-hydroxy-4,7,10-trioxa-undecanyl)]-1,4,7,-triscarboxymethyl-1,4,7,10-tetraazacyclododecan

Analog zu Beispiel 4b wurde die Titelverbindung aus Beispiel 9c anstelle von der Titelverbindung Beispiel 4a zur Komplexierung eingesetzt.
Ausbeute: 97% d. Th. eines farblosen, amorphen Pulvers

| Analyse (bezogen auf wasserfreie Substanz): | | | | |
|---|---|---|---|---|
| C 41,57 | H 6,35 | N 6,93 | Gd 19,44 | ber. |
| C 41,48 | H 6,41 | N 6,91 | Gd 19,37 | |

### Beispiel 10

### a) 9,14-Dioxa-docos(11)en

Analog zu Beispiel 9a wurde n-Octanol anstelle von Diethylenglycolmonomethylether eingesetzt.
Ausbeute: 59 % d. Th. eines farblosen Öls

| Analyse (bezogen auf wasserfreie Substanz): | | |
|---|---|---|
| C 76,86 | H 12,90 | ber. |
| C 76,78 | H 12,84 | |

### b) 11,12-Epoxy-9,14-dioxa-docosan

Analog zu Beispiel 9b wurde die Titelverbindung aus Beispiel 10a anstelle der Titelverbindung 9a eingesetzt.
Ausbeute: 83 % d. Th. eines farblosen Öls

| | | |
|---|---|---|
| C 73,12 | H 12,27 | ber. |
| C 73,04 | H 12,34 | |

### c) 10-[1-(2-oxadecyl)-2-hydroxy-4-oxa-dodecyl]-1,4,7-triscarboxymethyl-1,4,7,10-tetraazacyclododecan

Analog zu Beispiel 4a wurde 11,12-Epoxy-9,14-dioxa-docosan anstelle des in Beispiel 4a benutzten Epoxids eingesetzt.
Ausbeute: 37 % d. Th. eines farblosen Feststoffes

| Analyse (bezogen auf wasserfreie Substanz): | | | |
|---|---|---|---|
| C 60,51 | H 9,86 | N 8,30 | ber. |
| C 60,46 | H 9,94 | N 8,25 | |

### d) Gadolinium-Komplex von 10-[1-(2-oxadecyl)-2-hydroxy-4-oxa-dodecyl]-1,4,7-tris-carboxymethyl-1,4,7,10-tetraazacyclododecan

Analog zu Beispiel 4b wurde die Titelverbindung aus Beispiel 10c anstelle von der Titelverbindung 4a zur Komplexierung eingesetzt.
Ausbeute: 96 % d. Th. eines farblosen Pulvers

| Analyse (bezogen auf wasserfreie Substanz): | | | | |
|---|---|---|---|---|
| C 49,25 | H 7,66 | N 6,76 | Gd 18,97 | ber. |
| C 49,17 | H 7,80 | N 6,68 | Gd 18,91 | |

### Beispiel 11

### a) 10-(2-Hydroxy-5-phenyl-4-oxa-pentyl)-1,4,7-tris(carboxymethyl)-1,4,7,10-tetraazacyclododecan

10,2 g (29,45 mmol) 1,4,7-Triscarboxymethyl-1,4,7,10-tetraazacyclododecan (= DO3A) und 8,7 g (53,0 mmol) Benzyl-2,3-epoxypropylether werden in einer Mischung aus 50 ml Dioxan und 80 ml Wasser gelöst und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert-Buthylmethylether. Die wässrige Lösung wird mit 5N Salzsäure auf pH 3 gestellt und zur Trockene eingedampft. Der Rückstand wird im Vakuum eingeengt und mit 200 ml Methanol/50 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser gelöst und anschließend auf eine Saule aus Poly-(4-vinylpyridin) gegeben. Das Produkt wird mit einer Lösung aus Ethanol/Wasser= 1:5 eluiert. Nach Eindampfen im Vakuum erhält man 11,58 g (77 % d. Th.) der Titelverbindung als stark hygroskopischen, glasigen Feststoff.

| Analyse (bezogen auf wasserfreie Substanz): | | | |
|---|---|---|---|
| C 56,46 | H 7,50 | N 10,97 | ber. |
| C 56,41 | H 7,58 | N 10,81 | |

### b) Gadolinium-Komplex von 10-(2-Hydroxy-5-phenyl-4-oxa-pentyl)-1,4,7-tris(carboxymethyl)-1,4,7,10-tetraazacyclododecan

10,0 g (19,59 mmol) der Titelverbindung aus Beispiel 11a werden in 70 ml entionisiertem Wasser gelöst und 3,55g (9,79 mmol) Gadoliniumoxid zugesetzt. Man erwärmt 3 Stunden auf 90°C. Die abgekühlte Lösung wird mit je 3 ml saurem lonenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 12,5 g (96 % d. Th.) eines amorphen Pulvers

| Analyse (bezogen auf wasserfreie Substanz): | | | | |
|---|---|---|---|---|
| C 43,36 | H 5,31 | N 8,43 | Gd 23,65 | ber. |
| C 43,31 | H 5,37 | N 8,41 | Gd 23,58 | |

### c) ¹⁵¹Europium-Komplex von 10-(2-Hydroxy-5-phenyl-4-oxa-pentyl)-1,4,7-tris(carboxymethyl)-1,4,7,10-tetraazacyclododecan

Analog zu Beispiel 11b wurde ¹⁵¹Europiumoxid anstelle von Gadoliniumoxid eingesetzt.
Ausbeute: 95 % d. Th. eines amorphen Pulvers

| Analyse (bezogen auf wasserfreie Substanz): | | | | |
|---|---|---|---|---|
| C 42,79 | H 5,15 | N 8,68 | Eu 23,54 | ber. |
| C 42,72 | H 5,10 | N 8,73 | Eu 23,48 | |

### d) Ytterbium-Komplex von 10-(2-Hydroxy-5-phenyl-4-oxa-pentyl)-1,4,7,tris(carboxymethyl)-1,4,7,10-tetraazacyclododecan

Analog zu Beispiel 11b wurde Ytterbiumoxid anstelle von Gadoliniumoxid eingesetzt.
Ausbeute: 98 % d. Th. eines amorphen Pulvers

| Analyse (bezogen auf wasserfreie Substanz): | | | | |
|---|---|---|---|---|
| C 41,44 | H 4,99 | N 8,41 | Yb 25,96 | ber. |
| C 41,37 | H 5,05 | N 8,37 | Yb 25,91 | |

### Beispiel 12

### a) 10-[3-(4-Nitrophenoxy)-2-hydroxypropyl]-1,4,7-tris(carboxymethyl)-1,4,7,10-tetraazacyclododecan

12,68 g (64,95 mmol) 4-Nitrophenyl-2,3 epoxypropylether und 12,5 g (36,08 mmol) 1,4,7-Triscarboxymethyl-1,4,7,10-tetraazacyclododecan (= DO3A) werden in einer Mischung aus 70 ml Dioxan und 80 ml Wasser gelöst, und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml Essigsäureethylester. Die wässrige Lösung wird mit 5N Salzsäure auf pH 3 gestellt und zur Trockene eingedampft. Der Rückstand wird im Vakuum eingeengt und mit 200 ml Methanol/50 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 50 ml Wasser/30 ml Ethanol gelöst und anschließend auf eine Säule aus Poly-(4-vinylpyridin) gegeben. Das Produkt wird mit Ethanol/Wasser= 1:4 eluiert. Nach Eindampfen im Vakuum erhält man 14,43 g (74 % d. Th.) der Titelverbindung als stark hygroskopischen, glasigen Feststoff.

| Analyse (bezogen auf wasserfreie Substanz): | | | |
|---|---|---|---|
| C 51,11 | H 6,34 | N 12,96 | ber. |
| C 51,07 | H 6,41 | N 12,90 | |

### b) Gadolinium-Komplex von 10-[3-(4-Nitrophenoxy)-2-hydroxypropyl]-1,4,7-tris(carboxymethyl)-1,4,7,10-tetraazacyclododecan

14,1 g (26,08 mmol) der Titelverbindung aus Beispiel 12a werden in 100 ml entionisiertem Wasser gelöst und 4,73 g (13,04 mmol) Gadoliniumoxid zugesetzt. Man rührt 3 Stunden bei 90°C. Die abgekühlte Lösung wird mit je 3 ml saurem Ionenaustauscher (IR 120) und 3 ml basischem Austauscher (IRA 410) eine Stunde gerührt. Man filtriert vom Austauscher und dampft im Vakuum zur Trockene ein.
Ausbeute: 17,4 g (96 % d. Th.) eines glasigen Feststoffes

| Analyse (bezogen auf wasserfreie Substanz): | | | | |
|---|---|---|---|---|
| C 39,76 | H 4,50 | N 10,08 | Gd 22,63 | ber. |
| C 39,70 | H 4,53 | N 10,12 | Gd 22,57 | |

### c) Gadolinium-Komplex von 10-[3-(4-Aminophenoxy)-2-hydroxypropyl]-1,4,7-tris(carboxymethyl)-1,4,7,10-tetraazacyclododecan

15,0 g (21,59 mmol) der Titelverbindung aus Beispiel 12b werden in 250 ml entionisiertem Wasser gelöst und 3 g Palladium-Katalysator (10 % Pd auf Aktivkohle) zugesetzt. Man hydriert 2 Stunden bei 40°C (laut HPLC tritt vollständiger Umsatz ein). Man filtriert vom Katalysator ab und dampft im Vakuum zur Trockene ein.
Ausbeute: 14,07 g (98 % d. Th.) eines glasigen Feststoffes

| Analyse (bezogen auf wasserfreie Substanz): | | | | |
|---|---|---|---|---|
| C 41,55 | H 5,00 | N 10,53 | Gd 23,65 | ber. |
| C 41,50 | H 5,09 | N 10,49 | Gd 23,58 | |

### d) Gadolinium-Komplex von 10-[3-(4-Isothiocyanatophenoxy)-2-hydroxypropyl]-1,4,7-tris(carboxymethyl)-1,4,7,10-tetraazacyclododecan

10,0 g (15,04 mmol) der Titelverbindung aus Beispiel 12c und 30 ml Polyvinylpyridin (Reillex) werden in 200 ml entionisiertem Wasser gelöst und 5,19 g (45,13 mmol) Thiophosgen (in 150 ml Chloroform) zugesetzt. Man rührt 10 Minuten bei 40°C, dann 1 Stunde bei Raumtemperatur. Die zweiphasige Lösung wird vom Reillex abfiltriert und dann die organische Phase abgetrennt. Die wässrige Phase wird 2 mal mit 100 ml Chloroform extrahiert und anschließend gefriergetrocknet.
Ausbeute: 10,31 g (97 % d. Th.) eines weißen amorphen Pulvers

| Analyse (bezogen auf wasserfreie Substanz): | | | | | |
|---|---|---|---|---|---|
| C 40,78 | H 4,42 | N 9,91 | Gd 22,25 | S 4,54 | ber. |
| C 40,67 | H 4,51 | N 9,85 | Gd 22,19 | S 4,48 | |

### Beispiel 13

### a) 10-(2-Hydroxy-3-phenoxy-propyl)-1,4,7-tris(carboxymethyl)-1,4,7,10-tetraazacyclododecan

7,8 g (51,96 mmol) Phenyl-2,3-epoxypropylether und 10 g (28,87 mmol) 1,4,7-Triscarboxymethyl-1,4,7,10-tetraazacyclododecan (= DO3A) werden in einer Mischung aus 50 ml Dioxan und 80 ml Wasser gelöst und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert-Butylmethylether. Die wässrige Lösung wird mit 5N Salzsäure auf pH3 gestellt und zur Trockene eingedampft. Der Rückstand wird im Vakuum eingeengt und mit 200 ml Methanol/80 ml Methylenchlorid ausgekocht (extrahiert). Man kühlt im Eisbad ab und fitriert vom ausgefallenen Kaliumchlorid ab. Das Filtrat wird im Vakuum eingedampft, der Rückstand in 50 ml Wasser/20 ml Ethanol gelöst und anschließend auf eine Säule aus Poly-(4-vinylpyridin) gegeben. Das Produkt wird mit einer Lösung aus Ethanol/Wasser= 1:3 eluiert. Nach Eindampfen im Vakuum erhält man 8,17g (57 % d. Th.) der Titelverbindung als stark hygroskopischen, glasigen Feststoff.

| Analyse (bezogen auf wasserfreie Substanz): | | | |
|---|---|---|---|
| C 55,63 | H 7,31 | N 11,28 | ber. |
| C 55,59 | H 7,38 | N 11,24 | |

### b) Gadolinium-Komplex von 10-(2-Hydroxy-3-phenoxy-propyl)-1,4,7-tris(carboxymethyl)-1,4,7,10-tetraazacyclododecan

7,8 g (15,71 mmol) der Titelverbindung aus Beispiel 13a werden in 50 ml entionisiertem Wasser gelöst und 2,85 g (7,85 mmol) Gadoliniumoxid zugesetzt. Man erwärmt 3 Stunden auf 90°C. Die abgekühlte Lösung wird mit je 3 ml saurem Ionenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 9,71 g (95 % d. Th.) eines farblosen, amorphen Pulvers

| Analyse (bezogen auf wasserfreie Substanz): | | | | |
|---|---|---|---|---|
| C 42,45 | H 5,11 | N 8,61 | Gd 24,16 | ber. |
| C 42,38 | H 5,19 | N 8,56 | Gd 24,09 | |

### Beispiel 14

### a) 10-[2-Hydroxy-3-(4-methoxyphenoxy)-propyl]-1,4,7-tris(carboxymethyl)-1,4,7,10-tetraazacyclododecan

9,36 g (51,96 mmol) 4-Methoxy-phenyl-2,3-epoxypropylether und 10 g (51,96 mmol) 1,4,7-Triscarboxymethyl-1,4,7,10-tetraazacyclododecan (= DO3A) werden in einer Mischung aus 50 ml Dioxan und 80 ml Wasser gelöst und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert-Butyl-methylether. Die wässrige Lösung wird mit 5N Salzsäure auf pH 13 gestellt und zur Trockene eingedampft. Der Rückstand wird im Vakuum eingeengt und mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyrridin) gegeben. Das Produkt wird mit einer Lösung aus Ethanol/Wasser= 1:3 erluiert. Nach Eindampfen im Vakuum erhält man 9,27 g (61 % d. Th.) der Titelverbindung als stark hygroskopischen, glasigen Feststoff.

| Analyse (bezogen auf wasserfreie Substanz): | | | |
|---|---|---|---|
| C 54,74 | H 7,27 | N 10,46 | ber. |
| C 54,71 | H 7,30 | N 10,57 | |

### b) Gadolinium-Komplex von 10-[2-Hydroxy-3-(4-methoxyphenoxy)-propyl]-1,4,7-tris(carboxymethyl)-1,4,7,10-tetraazacyclododecan

8,8 g (16,71 mmol) der Titelverbindung aus Beispiel 14a werden in 60 ml entionisiertem Wasser gelöst und 3,03 g (8,36 mmol) Gadoliniumoxid zugesetzt. Man erwärmt 3 Stunden auf 90°C. Die abgekühlte Lösung wird mit je 3 ml saurem Ionenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 11,04g (97 % d. Th.) eines farblosen, amorphen Pulvers

| Analyse (bezogen auf wasserfreie Substanz): | | | | |
|---|---|---|---|---|
| C 42,34 | H 5,18 | N 8,23 | Gd 23,10 | ber. |
| C 42,28 | H 5,23 | N 8,17 | Gd 23,03 | |

### Beispiel 15

### a) 6,7-Epoxy-4-oxa-1-[4-(methoxy)-phlenyl]-heptan

Analog zu Beispiel 6b wurde 3-(4-Methoxyphenyl)-propan-1-ol anstelle der Titelverbindung aus Beispiel 6a eingesetzt.
Ausbeute: 38 % d. Th. eines farblosen, zähen Öls.

| | | |
|---|---|---|
| C 70,24 | H 8,16 | ber. |
| C 70,31 | H 8,20 | |

### b) 10-[2-Hydroxy-4-oxa-7-(4-methoxyphenyl)-heptyl]-1,4,7-tris(carboxymethyl)-1,4,7,10-tetraazacyclododecan

10,93 g (51,96 mmol) 6,7-Epoxy-4-oxa-1-[4-(methoxy)phenyl]heptan und 10 g (28,87 mmol) 1,4,7-triscarboxymethyl-1,4,7,10-tetraazacyclododecan (= DO3A) werden in einer Mischung aus 60 ml Dioxan und 90 ml Wasser gelöst, und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert.-Butyl-methylether. Die wässrige Lösung wird mit 5N Salzsäure auf pH 3 gestellt und zur Trockene eingedampft. Der Rückstand wird im Vakuum eingeengt und mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Mischung aus Ethanol/Wasser= 1:3 eluiert. Nach Eindampfen im Vakuum erhält man 9,69 g (59 % d. Th.) der Titelverbindung als stark hygroskopischen, glasigen Feststoff.

| Analyse (bezogen auf wasserfreie Substanz): | | | |
|---|---|---|---|
| C 57,03 | H 7,80 | N 9,85 | ber. |
| C 57,10 | H 7,85 | N 9,79 | |

### c) Gadolinium-Komplex von 10-[2-Hydroxy-4-oxa-7-(4-methoxyphenyl)-heptyl]-1,4,7-tris(carboxymethyl)-1,4,7,10-tetraazacyclododecan

9,2 g (16,18 mmol) der Titelverbindung aus Beispiel 15b werden in 80 ml entionisiertem **Wasser gelöst** und 2,93 g (8,09 mmol) Gadoliniumoxid zugesetzt. Man erwärmt 3 Stunden auf 90°C. Die abgekühlte Lösung wird mit je 3 ml saurem lonenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 11,34 g (97 % d. Th.) eines farblosen, amorphen Pulvers

| Analyse (bezogen auf wasserfreie Substanz): | | | | |
|---|---|---|---|---|
| C 44,86 | H 5,72 | N 7,75 | Gd 21,75 | ber. |
| C 44,79 | H 5,81 | N 7,69 | Gd 21,68 | |

### Beispiel 16

### a) 10-[3-(4-Chlorophenoxy)-2-hydroxypropyl]-1,4,7,tris(carboxymethyl)-1,4,7,10-tetraazacyclododecan

9,59 g (21,96 mmol) 4-Chlorophenyl-2,3-epoxypropylether und 10g (28,87 mmol) 1,4,7-triscarboxymethyl-1,4,7,10-tetraazacyclododecan (= DO3A) werden in einer Mischung aus 50 ml Dioxan und 80 ml Wasser gelöst und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert-Butyl-methylether. Die wässrige Lösung wird mit 5N Salzsäure auf pH 3 gestellt und zur Trockene eingedampft. Der Rückstand wird im Vakuum eingeengt und mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser= 1:3 eluiert. Nach Eindampfen im Vakuum erhält man 8,28 g (54 % d. Th.) der Titelverbindung als stark hygroskopischen, glasigen Feststoff.

| Analyse (bezogen auf wasserfreie Substanz): | | | | |
|---|---|---|---|---|
| C 52,02 | H 6,64 | N 10,55 | Cl 6,68 | ber. |
| C 52,05 | H 6,71 | N 10,49 | Cl 6,60 | |

### b) Gadolinium-Komplex von 10-[3-(4-Chlorophenoxy)-2-hydroxypropyl]-1,4,7,tris(carboxymethyl)-1,4,7,10-tetraazacyclododecan

7,9 g (14,88 mmol) der Titelverbindung aus Beispiel 16a werden in 60 ml entionisiertem Wasser gelöst und 2,70 g (7,44 mmol) Gadoliniumoxid zugesetzt. Man erwärmt 3 Stunden auf 90°C. Die abgekühlte Lösung wird mit je 3 ml saurem Ionenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 9,79 g (96 % d. Th.) eines farblosen, amorphen Pulvers

| Analyse (bezogen auf wasserfreie Substanz): | | | | | |
|---|---|---|---|---|---|
| C 40,32 | H 4,71 | N 8,18 | Gd 22,95 | Cl 5,17 | ber. |
| C 40,27 | H 4,80 | N 8,09 | Gd 22,91 | Cl 5,10 | |

### Beispiel 17

### a) 2,3-Epoxy-1-[4-(5-hydroxypentyl)-phenoxy]-propan

Analog zu Beispiel 6b wurde 5-(4-Hydroxyphenyl)-pentan-1-ol anstelle der Titelverbindung aus Beispiel 6a eingesetzt.
Ausbeute: 51 % d. Th. eines farblosen Öls

| Analyse (bezogen auf wasserfreie Substanz): | | |
|---|---|---|
| C 71,16 | H 8,53 | ber. |
| C 71,08 | H 8,62 | |

### b) 10-[2-Hydroxy-3-(4-(5-hydroxypentyl)-phenoxy)-propyl]-1,4,7-tris(carboxymethyl)-1,4,7,10-tetraazacyclododecan

12,3 g (51,97 mmol) 2,3-Epoxy-1-[4-(5-hydroxypentyl)-phenoxy]-propan und 10 g (28,87 mmol) 1,4,7-triscarboxymethyl-1,4,7,10-tetraazacyclododecan (= DO3A) werden in einer Mischung aus 50 ml Dioxan und 80 ml Wasser gelöst, und der pH-Wert mit 6N Kalilauge auf pH 10 gebracht. Man rührt 24 Stunden bei 40°C. Man dampft zur Trockene ein, nimmt den Rückstand mit 200 ml Wasser/50ml Methanol auf und extrahiert 2 mal mit 100 ml tert-Butyl-methylether. Die wässrige Lösung wird mit 5N Salzsäure auf pH 3 gestellt und zur Trockene eingedampft. Der Rückstand wird im Vakuum eingeengt und mit 200 ml Methanol/50 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 50 ml Wasser/20 ml Ethanol gelöst und anschließend auf eine Säule aus Poly(4-vinylpyridin) gegeben. Das Produkt wird mit einer Lösung aus Ethanol/Wasser eluiert. Nach Eindampfen im Vakuum erhält man 8,92 g (53 % d. Th.) der Titelverbindung als stark hygroskopischen, glasigen Feststoff.

| Analyse (bezogen auf wasserfreie Substanz): | | | |
|---|---|---|---|
| C 57,72 | H 7,72 | N 9,62 | ber. |
| C 57,68 | H 7,79 | N 9,54 | |

### c) Gadolilnium-Komplex von 10-[2-Hydroxy-3-(4-(5-hydroxypentyl)-phenoxy)-propyl]-1,4,7-tris(carboxymethyl)-1,4,7,10-tetraazacyclododecan

8,7 g (14,93 mmol) der Titelverbindung aus Beispiel 17b werden in 70 ml entionisiertem Wasser gelöst und 2,71 g (7,47 mmol) Gadoliniumoxid zugesetzt. Man erwärmt 3 Stunden auf 90°C. Die abgekühlte Lösung wird mit je 3 ml saurem lonenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 10,67 g (97% d. Th.) eines farblosen, amorphen Pulvers

| Analyse (bezogen auf wasserfreie Substanz): | | | | |
|---|---|---|---|---|
| C 45,64 | H 5,88 | N 7,60 | Gd 21,34 | ber. |
| C 45,60 | H 5,93 | N 7,54 | Gd 21,28 | |

### Beispiel 18

### a) 10-[3-(2,6-Dimethylphenoxy)-2-hydroxypropyl]-1,4,7-tris(carboxymethyl)-1,4,7,10-tetraazacyclododecan

9,59 g (51,96 mmol) 2,6-Dimethyl-phenyl-2,3-epoxypropylether und 10 g (28,87 mmol) 1,4,7-triscarboxymethyl-1,4,7,10-tetraazacyclododecan (= DO3A) werden in einer Mischung aus 50 ml Dioxan und 80 ml Wasser gelöst und der pH-Wert mit 6N Kalilauge auf pH 10 gebracht. Man rührt 24 Stunden bei 40°C. Man dampft zur Trockene ein, nimmt den Rückstand mit 200 ml Wasser/50 ml Methanol auf und extrahiert 2 mal mit 100 ml tert-Butyl-methylether. Die wässrige Lösung wird mit 5N Salzsäure auf pH 3 gestellt und zur Trockene eingedampft. Der Rückstand wird mit 200 ml Methanol/80 80ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 50 ml Wasserund 30 ml Ethanol gelöst und anschließend auf eine Säule aus Poly-(4-vinylpyridin) gegeben. Das Produkt wird mit einer Lösung aus Ethanol/Wasser= 1:3 eluiert. Nach Eindampfen im Vakuum erhält man 9,24g (61 % d. Th.) der Titelverbindung als stark hygroskopischen, glasigen Feststoff.

| Analyse (bezogen auf wasserfreie Substanz): | | | |
|---|---|---|---|
| C 57,24 | H 7,68 | N 10,68 | ber. |
| C 57,18 | H 7,74 | N 10,64 | |

### b) Gadolinium-Komplex von 10-[3-(2,6-Dimethylphenoxy)-2-hydroxypropyl]-1,4,7-tris(carboxymethyl)-1,4,7,10-tetraazacyclododecan

9,0 g (17,16 mmol) der Titelverbindung aus Beispiel 18a werden in 70 ml entionisiertem Wasser gelöst und 3,11 g (8,58 mmol) Gadoliniumoxid zugesetzt. Man erwärmt 3 Stunden auf 90°C. Die abgekühlt Lösung wird mit je 3 ml saurem Ionenaustauscher (IR 120) und 3 ml basischem Austauscher (IRA 410) eine Stunde bei Raumtemperatur gerührt. Nach Filtration vom Austauscher wird das Filtrat gefriergetrocknet.
Ausbeute: 11,30 g (97 % d. Th.) eines farblosen, amorphen Pulvers

| Analyse (bezogen auf wasserfreie Substanz): | | | | |
|---|---|---|---|---|
| C 44,23 | H 5,49 | N 8,25 | Gd 23,16 | ber. |
| C 44,18 | H 5,54 | N 8,20 | Gd 23,08 | |

### Beispiel 19

### a) 2,3-Epoxy-1-[4-(exthoxycarbonylmethyl)-phenoxy]-propan

Analog zu Beispiel 6b wurde 4-Hydroxyphenylessigsäureethylester anstelle der Titelverbindung aus Beispiel 6a eingesetzt.
Ausbeute: 61 % d. Th. eines farblosen zähen Öls.

| Analyse (bezogen auf wasserfreie Substanz): | | |
|---|---|---|
| C 66,09 | H 6,83 | ber. |
| C 66,14 | H 6,74 | |

### b) 10-[2-Hydroxy-3-(4-(carboxymethyl)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonylmethyl)-phenoxy]-propan werden in 80 ml Dioxan/60 ml Wasser gegeben und mit 6N Kalilauge auf pH 13 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 8,32 g (52 % d. Th.) eines glasigen Feststoffes

| Analyse (bezogen auf wasserfreie Substanz): | | | |
|---|---|---|---|
| C 54,14 | H 6,91 | N 10,10 | ber. |
| C 54,06 | H 6,98 | N 10,06 | |

### c) Gadolinium-Komplex von 10-[2-Hydroxy-3-(4-(carboxymethyl)-phenoxy)-propyl]-1,4,7-tris(carboxymethyl)-1,4,7,10-tetraazacyclododecan (als Natriumsalz)

8,1 g (14,61 mmol) der Titelverbindung aus Beispiel 19b werden in 60 ml entionisiertem Wasser gelöst und 2,65 g (7,30 mmol) Gadoliniumoxid zugesetzt. Es wird 3 Stunden auf 90°C erhitzt. Die abgekühfte Lösung wird eine Stunde mit 7 ml schwach saurem Ionenaustauscher (AMB252c) bei Raumtemperatur gerührt. Man filtriert vom Austauscher ab. Das Filtrat wird mit 2N Natronlauge auf pH7,2 eingestellt und gefriergetrocknet.
Ausbeute: 10,14 g (95 5 d. Th.) eines farblosen, amorphen Pulvers

| Analyse (bezogen auf wasserfreie Substanz): | | | | | |
|---|---|---|---|---|---|
| C 41,09 | H 4,69 | N 7,67 | Gd 21,52 | Na 3,15 | ber. |
| C 41,04 | H 4,78 | N 7,61 | Gd 21,47 | Na 3,19 | |

### Beispiel 20

### a) 2,3-Epoxy-1-[4-(ethoxycarbonyl)-cyclohexyloxy]-propan

Analog zu Beispiel 6b wurde 4-Hydroxy-Cyclohexan-carbonsäureethylester anstelle der Titelverbindung aus Beispiel 6a eingesetzt.
Ausbeute: 33 % d. Th. eines farblosen Öls

| Analyse (bezogen auf wasserfreie Substanz): | | |
|---|---|---|
| C 63,14 | H 8,33 | ber. |
| C 63,07 | H 8,91 | |

### b) 10-[2-Hydroxy-3-(4-(carboxy)-cyclohexyloxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(ethoxycarbonyl)-cyclohexyloxy]-propan werden in 80 ml Dioxan/60 ml Wasser gegeben und mit 6N Kalilauge auf pH 13 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 8,2g (52 % d. Th.) eines glasigen Feststoffes

| Analyse (bezogen auf wasserfreie Substanz): | | | |
|---|---|---|---|
| C 52,74 | H 7,74 | N 10,25 | ber. |
| C 52,68 | H 7,81 | N 10,19 | |

### c) Gadolinium-Komplex von 10-[2-Hydroxy-3-(4-(carboxy)-cyclohexyloxy)-propyl]-1,4,7-tris(carboxymethyl)-1,4,7,10-tetraazacyclododecan (als Natriumsalz)

8,0 g (14,64 mmol) der Titelverbindung aus Beispiel 20b werden in 70 ml entionisiertem Wasser gelöst und 2,66g (7,32 mmol) Gadoliniumoxid zugesetzt. Es wird 3 Stunden auf 90°C erhitzt. Die abgekühfte Lösung wird eine Stunde mit 10 ml schwach saurem Ionenaustauscher (AMB 252c) bei Raumtemperatur gerührt. Man filtriert vom Austauscher ab. Das Filtrat wird mit 2N Natronlauge auf pH 7,2 eingestellt und gefriergetrocknet.
Ausbeute: 9,84 g (93 % d. Th.) eines farblosen, amorphen Pulvers

| Analyse (bezogen auf wasserfreie Substanz): | | | | | |
|---|---|---|---|---|---|
| C 39,88 | H 5,30 | N 7,75 | Gd 21,75 | Na 3,13 | ber. |
| C 39,81 | H 5,37 | N 7,69 | Gd 21,70 | Na 3,25 | |

### Beispiel 21

### a) 2,3-Epoxy-1-[4-(2-ethoxycarbonylethyl)-phenoxy]-propan

Analog zu Beispiel 6b wurde 2-(4-Hydroxyphenyl)-propansäureethylester anstelle der Titelverbindung aus Beispiel 6a eingesetzt.
Ausbeute: 67 % d. Th. eines farblosen Öls

| Analyse (bezogen auf wasserfreie Substanz): | | |
|---|---|---|
| C 67,18 | H 7,25 | ber. |
| C 67,09 | H 7,32 | |

### b) 10-[2-Hydroxy-3-(4-(2-carboxyethyl)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonylethyl)-phenoxy]-propan werden in 80 ml Dioxan/60 ml Wasser gegeben und mit 6N Kalilauge auf pH 14 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 7,88g (48 % d. Th.) eines glasigen Feststoffes

| Analyse (bezogen auf wasserfreie Substanz): | | | |
|---|---|---|---|
| C 54,92 | H 7,09 | N 9,85 | ber. |
| C 54,87 | H 7,15 | N 9,79 | |

### c) Gadolinium-Komplex von 10-[2-Hydroxy-3-(4-(2-carboxyethyl)-phenoxy)-propyl]-1,4,7-tris(carboxymethyl)-1,4,7,10-tetraazacyclododecan (als Natriumsalz)

7,5 g (13,19 mmol) der Titelverbindung aus Beispiel 21b werden in 50 ml entionisiertem Wasser gelöst und 2,39 g (6,59 mmol) Gadoliniumoxid zugesetzt. Es wird 3 Stunden auf 90°C erhitzt. Die abgekühlte Lösung wird eine Stunde mit 7 ml schwach saurem Ionenaustauscher (AMB 252c) bei Raumtemperatur gerührt. Man filtriert vom Austauscher ab. Das Filtrat wird mit 2N Natronlauge auf pH 7,2 eingestellt und gefriergetrocknet.
Ausbeute: 9,23 g (94 % d. Th.) eines farblosen, amorphen Pulvers

| Analyse (bezogen auf wasserfreie Substanz): | | | | | |
|---|---|---|---|---|---|
| C 41,93 | H 4,87 | N 7,52 | Gd 21,11 | Na 3,09 | ber. |
| C 41,87 | H 4,93 | N 7,47 | Gd 21,06 | Na 3,14 | |

### Beispiel 22

### a) 2,3-Epoxy-1-[4-(2-ethoxycarbonyl-1-oxa-ethyl)-phenoxy]-propan

Analog zu Beispiel 6b wurde 4-Hydroxy-phenoxy-essigsäureethylester anstelle der Titelverbindung aus Beispiel 6a eingesetzt.
Ausbeute: 57 % d. Th. eines farblosen Öls

| Analyse (bezogen auf wasserfreie Substanz): | | |
|---|---|---|
| C 61,89 | H 6,39 | ber. |
| C 61,78 | H 6,43 | |

### b) 10-[2-Hydroxy-3-(4-(2-carboxy-1-oxa-ethyl)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonylethyl)-phenoxy]-propan werden in 80 ml Dioxan/60 ml Wasser gegeben und mit 6N Kalilauge auf pH 13 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedamft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 8,40 g (51 % d. Th.) eines glasigen Feststoffes

| Analyse (bezogen auf wasserfreie Substanz): | | | |
|---|---|---|---|
| C 52,62 | H 6,71 | N 9,82 | ber. |
| C 52,58 | H 6,78 | N 9,77 | |

### b) Gadolinium-Komplex von 10-[2-Hydroxy-3-(4-(2-carboxy-1-oxa-ethyl)-phenoxy)-propyl]-1,4,7-tris(carboxymethyl)-1,4,7,10-tetraazacyclododecan (als Natriumsalz)

8,1 g (14,2 mmol) der Titelverbindung aus Beispiel 22b werden in 70 ml entionisiertem Wasser gelöst und 2,57 g (7,1 mmol) Gadoliniumoxid zugesetzt. Es wird 3 Stunden auf 90°C erhitzt. Die abgekühlte Lösung wird eine Stunde mit 8 ml schwach saurem Ionenaustauscher (AMB 252c) bei Raumtemperatur gerührt. Man filtriert vom Austauscher ab. Das Filtrat wird mit 2N Natronlauge auf pH 7,2 eingestellt und gefriergetrocknet.
Ausbeute: 9,86 g (93 % d. Th.) eines amorphen Pulvers

| Analyse (bezogen auf wasserfreie Substanz): | | | | | |
|---|---|---|---|---|---|
| C 40,21 | H 4,59 | N 7,50 | Gd 21,06 | Na 3,08 | ber. |
| C 40,16 | H 4,65 | N 7,47 | Gd 21,00 | Na 3,15 | |

### Beispiel 23

### a) 2,3-Epoxy-1-[4-(ethoxycarbonyl)-phenoxy]-propan

Analog zu Beispiel 6b wurde 4-Hydroxy-phenylcarbonsäureethylester anstelle der Titelverbindung aus Beispiel 6a eingesetzt.
Ausbeute: 73 % d. Th. eines farblosen Öls

| Analyse (bezogen auf wasserfreie Substanz): | | |
|---|---|---|
| C 64,85 | H 6,35 | ber. |
| C 64,78 | H 6,43 | |

### b) 10-[2-Hydroxy-3-(4-(carboxy)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonyl)-phenoxy]-propan werden in 80 ml Dioxan/60 ml Wasser gegeben und mit 6N Kalilauge auf pH 13 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 8,43 g (54 % d. Th.) eines glasigen Feststoffes

| Analyse (bezogen auf wasserfreie Substanz): | | | |
|---|---|---|---|
| C 53,32 | H 6,71 | N 10,36 | ber. |
| C 53,29 | H 6,81 | N 10,31 | |

### c) Gadolinium-Komplex von 10-[2-Hydroxy-3-(4-(carboxy)-phenoxy)-propyl]-1,4,7-tris(carboxymethyl)-1,4,7,10-tetraazacyclododecan (als Natriumsalz)

8,1 g (14,98 mmol) der Titelverbindung aus Beispiel 23b werden in 70 ml entionisiertem Wasser gelöst und 2,72 g (7,49 mmol) Gadoliniumoxid zugesetzt. Es wird 3 Stunden auf 90°C erhitzt. Die abgeküghlte Lösung wird eine Stunde mit 6 ml schwach saurem Ionenaustauscher (AMB 252c) bei Raumtemperatur gerührt. Man filtriert vom Austauscher ab. Das Filtrat wird mit 2N Natronlauge auf pH 7,2 eingestellt und gefriergetrocknet.
Ausbeute: 9,99 g (93 % d. Th.) eines farblosen amorphen Pulvers

| Analyse (bezogen auf wasserfreie Substanz): | | | | | |
|---|---|---|---|---|---|
| C 40,22 | H 4,50 | N 7,82 | Gd 21,94 | Na 3,21 | ber. |
| C 40,17 | H 4,60 | N 7,76 | Gd 21,88 | Na 3,27 | |

### Beispiel 24

### a) 2,3-Epoxy-1-[4-(ethoxycarbonyl)-benzyloxy]-propan

Analog zu Beispiel 6b wurde 4-Hydroxy-phenylessigsäureethylester anstelle der Titelverbindung aus Beispiel 6a eingesetzt.
Ausbeute: 53 % d. Th. eines farblosen Öls

| Analyse (bezogen auf wasserfreie Substanz): | | |
|---|---|---|
| C 66,09 | H 6,83 | ber. |
| C 66,16 | H 6,78 | |

### b) 10-[2-Hydroxy-3-(4-(carboxymethyl)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonyl)-benzyloxy]-propan werden in 80 ml Dioxan/60 ml Wasser gegeben und mit 6N Kalilauge auf pH 13 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 8,17 g (51 %d. Th.) eines glasigen Feststoffes

| Analyse (bezogen auf wasserfreie Substanz): | | |
|---|---|---|
| C 54,14 | H 6,91 | N 10,10 |
| C 54,09 | H 6,98 | N 10,02 |

### c) Gadolinium-Komplex von 10-[2-Hydroxy-3-(4-(carboxymethyl)-phenoxy)-propyl]-1,4,7-tris(carboxymethyl)-1,4,7,10-tetraazacycloclodecan (als Natriumsalz)

7,9 g (14,24 mmol) der Titelverbindung aus Beispiel 24b werden in 80 ml entionisiertem Wasser gelöst und 2,58 g (7,12 mmol) Gadoliniumoxid zugesetzt. Es wird 3 Stunden auf 90°C erhitzt. Die abgekühlte Lösung wird eine Stunde mit 8 ml schwach saurem Ionenaustauscher (AMB 252c) bei Raumtemperatur gerührt. Man filtriert vom Austauscher ab. Das Filtratird mit 2N Natronlauge auf pH 7,2 eingestellt und gefriergetrocknet.
Ausbeute: 9,58 g (92 % d. Th.) eines farblosen, amorphen Pulvers

| Analyse (bezogen auf wasserfreie Substanz): | | | | | |
|---|---|---|---|---|---|
| C 41,09 | H 4,69 | N 7,67 | Gd 21,52 | Na 3,15 | ber. |
| C 41,02 | H 4,74 | N 7,61 | Gd 21,47 | Na 3,21 | |

### Beispiel 25

### Bis-Palmitinsäureester vom Gadolinium-Komplex von 10-(1-Hydroxymethyl-2,3-dihydroxypropyl)-1,4,7-tris(carboxymethyl)-1,4,7,10-tetraazacyclododecan

5 g (8,28 mmol) des Gadolinium-Komplexes von 10-(1-Hydroxymethyl-2,3-dihydroxypropyl)-1,4,7-tris-(carboxymethyl)-1,4,7,10-tetraazacyclododecan (siehe Europäische Anmeldung Nr. 91250081.6) werden in 100 ml Dimethylformamid gelöst und 4,04 g (40 mmol) Triethylamin zugegeben. Innerhalb 15 Minuten werden 6,83g (24,83 mmol) Palmitinsäurechlorid zugetropft. Es wird 2 Tage bei Raumtemperatur gerührt. Die Lösung wird im Vakuum zur Trockene eingedampft und der Rückstand an RP-18 (Reversed Phase/Laufmittel: Gradiert Wasser/Acetonitril) chromatographiert.
Ausbeute: Nach Eindampfen der Hauptfraktionen erhält man 7,25 g (81 % d. Th.) eines wachsartigen Feststoffes.

| Analyse (bezogen auf wasserfreie Substanz): | | | | |
|---|---|---|---|---|
| C 55,53 | H 8,48 | N 5,18 | Gd 14,54 | ber. |
| C 55,61 | H 8,30 | N 5,25 | Gd 14,40 | |

### Beispiel 26

### a) 3-Hydroxy-tridec(12)en

Zu einer Ethylmagnesiumjodid-Lösung(hergestellt aus 2,92 g (120 mmol) Magnesium und 18,71 g (120 mmol) Ethyljodid in 50 ml Ether) tropft man bei 0°C eine Lösung von 16,83g (100 mmol) 10-Undecenaldehyd in 30 ml Ether zu und rührt anschließend 5 Stunden bei Raumtemperatur. Man tropft vorsichtig 100 ml Wasser zu und stellt die wässrige Phase mit 10%iger Salzsäure auf pH 2. Die organische Phase wird abgetrennt und die wässrige Phase 2 mal mit je 40 ml Ether extrahiert. Die vereinigten organischen Phasen werden über Magnesiumsultat getrocknet und im Vakuum eingedampft. Das zurückbleibende Öl wird an Kieselgel chromatographiert (Laufmittel: Hexan/Essigester= 20:1).
Ausbeute: 16,86 g (85 % d. Th.) eines farblosen Öls

| Analyse (bezogen auf wasser freie Substanz): | | |
|---|---|---|
| C 78,72 | H 13,21 | ber. |
| C 78,90 | H 13,12 | |

### b) 3-Benzyloxy-tridec(12)en

Zu 16 g (80,67 mmol) der Titelverbindung aus Beispiel 26a in 200 ml Dimethylformamid gibt man 2,52 g (104,87 mmol) Natriumhydrid und rührt 2 Stunden bei 50°C. Man kühlt im Eisbad auf 0°C und setzt 17,94 g (104,87 mmol) Benzylbromid zu. Anschließend wird über Nacht bei Raumtemperatur gerührt. Es werden 800 ml Wasser zugegeben und die Lösung 2 mal mit 200 ml Hexan extrahiert. Die vereinigten organischen Phasen werden über Magnesiumsulfat getrocknet und im Vakuum eingedampft.
Ausbeute: 23,03 g (99 % d. Th.) eines farblosen Öls

| Analyse (bezogen auf wasserfreie Substanz): | | |
|---|---|---|
| C 83,27 | H 11,18 | ber. |
| C 83,15 | H 11,25 | |

### c) 1,2-Epoxy-11-benzyloxy-tridecan

14,8 g (51,3 mmol) der Titelverbindung aus Beispiel 26b werden in 100 ml Chloroform gelöst. Bei 0°C setzt man 11,51 g (66,7 mmol) m-Chlorperoxybenzoesäure zu und läßt anschließend auf Raumtemperatur erwärmen. Man rührt 24 Stunden bei Raumtemperatur. Der ausgefallene Niederschlag wird abfiltriert und das Filtrat mehrmals mit konz. Natriumcarbonat-Lösung gewaschen. Die organische Phase wird über Magnesiumsulfat getrocknet und im Vakuum eingedampft. Der Rückstand wird an Kieselgel (Laufmittel: Hexar/Essigester 15:1) chromatographiert.
Ausbeute: 12,5 g (80 % d. Th.) eines farblosen Öls

| Analyse (bezogen auf wasserfreie Substanz): | | |
|---|---|---|
| C 78,90 | H 10,59 | ber. |
| C 78,77 | H 10,65 | |

### d) 10-(2-Hydroxy-11-benzyloxy-tridecyl)-1,4,7-tris-carboxymethyl-1,4,7,10-tetraazacyclododecan

Analog zu Beispiel 4a wurde die Titelverbindung aus Beispiel 26c anstelle des in Beispiel 4a benutzten Epoxids eingesetzt.
Ausbeute: 59 % d. Th. eines glasigen, hygroskopischen Feststoffes

| Analyse (bezogen auf wasserfreie Substanz): | | | |
|---|---|---|---|
| C 62,74 | H 8,98 | N 8,61 | ber. |
| C 62,58 | H 8,81 | N 8,75 | |

### e) Gadolinium-Komplex von 10-(2-Hydroxy-11-benzyloxy-tridecyl)-1,4,7-tris-carboxymethyl-1,4,7,10-tetraazacyclododecan

Analog zu Beispiel 4b wurde die Titelverbindung aus Beispiel 26d anstelle der Titelverbindung aus Beispiel 4a zur Komplexierung eingesetzt.
Ausbeute: 96 % d. Th. eines farblosen, amorphen Pulvers

| Analyse (bezogen auf wasserfreie Substanz): | | | |
|---|---|---|---|
| C 50,72 | H 6,89 | N 6,96 | ber. |
| C 50,60 | H 6,96 | N 6,64 | |

### Beispiel 27

### a) 1-[(4-Benzyloxy)-phenoxy]-undec(10)en

32,43 g (100 mmol) des p-Toluolsulfonsäureesters von 10-Undecenol, 20,03 g (100 mmol) 4-Benzyloxy-phenol und 5,61 g (100 mmol) feingepulvertes Kaliumhydroxid in 300 ml Toluol werden über Nacht bei 70°C gerührt. Man kühlt ab, gibt 500 ml 1N Natronlauge zu und trennt die organische Phase ab. Die wässrige Phase wird einmal mit 100 ml Toluol extrahiert, die vereinigten organischen Phasen über Magnesiumsulfat getrocknet und im Vakuum eingedampft. Der Rückstand wird aus wenig heißem Methanol umkristallisiert.
Ausbeute: 26,44 g (75 % d. Th.) farblose Blättchen

| Analyse (bezogen auf wasserfreie Substanz): | | |
|---|---|---|
| C 81,77 | H 9,15 | ber. |
| C 81,58 | H 9,27 | |

### b) 1,2-Epoxy-11-[(4-benzyloxy)-phenoxy]-undecan

Analog zu Beispiel 26c wird die Titelverbindung aus Beispiel 27a anstelle der Titelverbindung aus Beispiel 26b zur Epoxidierung eingesetzt.
Ausbeute: 87 % d. Th. eines kristallinen Feststoffes (aus Methanol)

| Analyse (bezogen auf wasserfreie Substanz): | | |
|---|---|---|
| C 78,22 | H 8,75 | ber. |
| C 78,05 | H 8,87 | |

### c) 10-[2-Hydroxy-11-(4-benzyloxy-phenoxy)-undecyl]-1,4,7-triscarboxymethyl-1,4,7,10-tetraazacyclododecan

Analog zu Beispiel 4a wurde die Titelverbindung aus Beispiel 27b anstelle des in Beispiel 4a benutzten Epoxids eingesetzt.
Ausbeute: 69 % d. Th. eines glasigen Feststoffes

| Analyse (bezogen auf wasserfreie Substanz): | | | |
|---|---|---|---|
| C 63,84 | H 8,18 | N 7,84 | ber. |
| C 63,67 | H 8,31 | N 7,60 | |

### d) Gadolinium-Komplex von 10-[2-Hydroxy-11-(4-benzyloxy-phenoxy)-undecyl]-1,4,7-triscarboxymethyl-1,4,7,10-tetraazacyclododecan

Analog zu Beispiel 4b wurde die Titelverbindung aus Beispiel 27c anstelle der Titelverbindung aus Beispiel 4a zur Komplexierung eingesetzt.
Ausbeute: 98 % d. Th. eines farblosen kristalllinen Pulvers

| Analyse (bezogen auf wasserfreie Substanz): | | | | |
|---|---|---|---|---|
| C 52,51 | H 6,38 | N 6,45 | Gd 18,09 | ber. |
| C 52,32 | H 6,45 | N 6,28 | Gd 17,91 | |

### Beispiel 28

### 10-[2-Hydroxy-11-(4-hydroxy-phenoxy)-undecyl]-1,4,7-triscarboxymethyl-1,4,7,10-tetraazacyclododecan

Analog zu Beispiel 7 wurde die Titelverbindung aus Beispiel 27d hydriert.
Ausbeute: 97% d. Th. eines glasigen Feststoffes

| Analyse (bezogen auf wasserfreie Substanz): | | | | |
|---|---|---|---|---|
| C 47,80 | H 6,34 | N 7,19 | Gd 20,19 | ber. |
| C 47,61 | H 6,50 | N 7,02 | Gd 20,03 | |

### Beispiele für die Darreichungsform

1) 372,42 g (0,5 mol) des in Beispiel 21c beschriebenen Komplexsalzes werden in 500 ml Wasser pro injectione (p.i.) unter Erwärmen gelöst. Nach Zugabe von 1,2 g Tromethamin wird die neutrale Lösung mit Wasser p.i. auf 1000 ml aufgefüllt. Die Lösung wird ultrafiltriert und in Flaschen abgefüllt. Nach Hitzesterilisation ist sie für die parenterale Applikation gebrauchsfertig.
2) 128 g (0,19 mol) des in Beispiel 14b beschriebenen Komplexes werden mit 120g Saccharose und 5 g Polyoxyethylenpolyoxypropylen unter Zugabe von 10 mg Himbeeraromastoff intensiv vermischt. Das erhaltene Pulver dient zur oralen Applikation.

| **T**_{**1**}**-Relaxivitäten (im Plasma) einiger ausgewählter Beispiele** | |
|---|---|
| Beispiel Nr. | T₁-Relaxivity (mM·s)^{-1*} |
| 1b | 7,30 |
| 2b | 7,27 |
| 3b | 7,16 |
| 4b | 24,47 |
| 5c | 12,18 |
| 6d | 24,16 |
| 7 | 9,91 |
| 8b | 5,76 |
| 11b | 8,66 |
| 12b | 10,23 |
| 13b | 8,70 |
| 14b | 7,25 |
| 21c | 7,28 |
| 23c | 7,04 |
| Magnevist® | 4,90 |
| Dotarem® | 4,30 |

| | |
|---|---|
| *) Die T₁-Relaxivity wurde mit einem NMR-Pulspektrometer (Minispec PC20) bei 20 Mhz (= 0,47 T) und 39°C in Human-Plasma gemessen | |

### Beispiel für eine in-vivo NMR-Diagnostik

Die Versuchstiere (Ratte, Wistar Han ♂, mit Novikoff Hepatom in der Leber) werden für die Kernspintomographische Untersuchung narkotisiert (Rompun® + Ketavet®) und zwecks Applikation des Kontrastmittels mit einem Katheter in der Schwanzvene versehen. Die Untersuchung erfolgt in einem MRI Experimentalgerät der Firma General Electric (Feldstärke 2 Tesla). Die Aufnahmen werden miteiner T₁-gewichteten Spin-Echo-Sequenz erstellt (TR = 400 m sec, TE = 20 m sec, 8 Mittelungen (averages) 3 mm Schichtdicke, axiale Schnittführung). Bild 1 zeigt die Leber mit dem Tumor vor Kontrastmittelgabe. Der Tumor ist mehr oder weniger isodens mit dem Leberparenchym und nicht von diesem abgrenzbar. 1 Minute nach i.v. Gabe von der Titelverbindung aus Beispiel 14b (0,1 mmol Gd/kg) weist die Leber eine erhöhte Signalintensität auf, während die Signalintensität des Tumors (rechts oben) sich nicht verändert hat, so daß beide jetzt sehr gut voneinander abzugrenzen sind (Bild 2). Auch 30 Minuten nach Applikation (Bild 3) ist der Kontrast zwischen Leber und Tumor noch vollständig erhalten. Das bedeutet, daß diese Substanz in Gegensatz zu den Handelspräparaten Magnevist® und Dotarem® in der Leber angereichert wird und hierdurch im Vergleich zu diesen bei gleicher Dosis erstens einen wesentlichen besseren Kontrast zwischen Leberparenchym und Tumor (und auch anderen pathologischen Erscheinungen) erzeugt und zweitens dieser Kontrast über längere Zeit erhalten bleibt. Letzteres ist von Vorteil, da hiermit eine größere Zeitspanne für eine erfolgreiche Diagnose zur Verfügung steht.

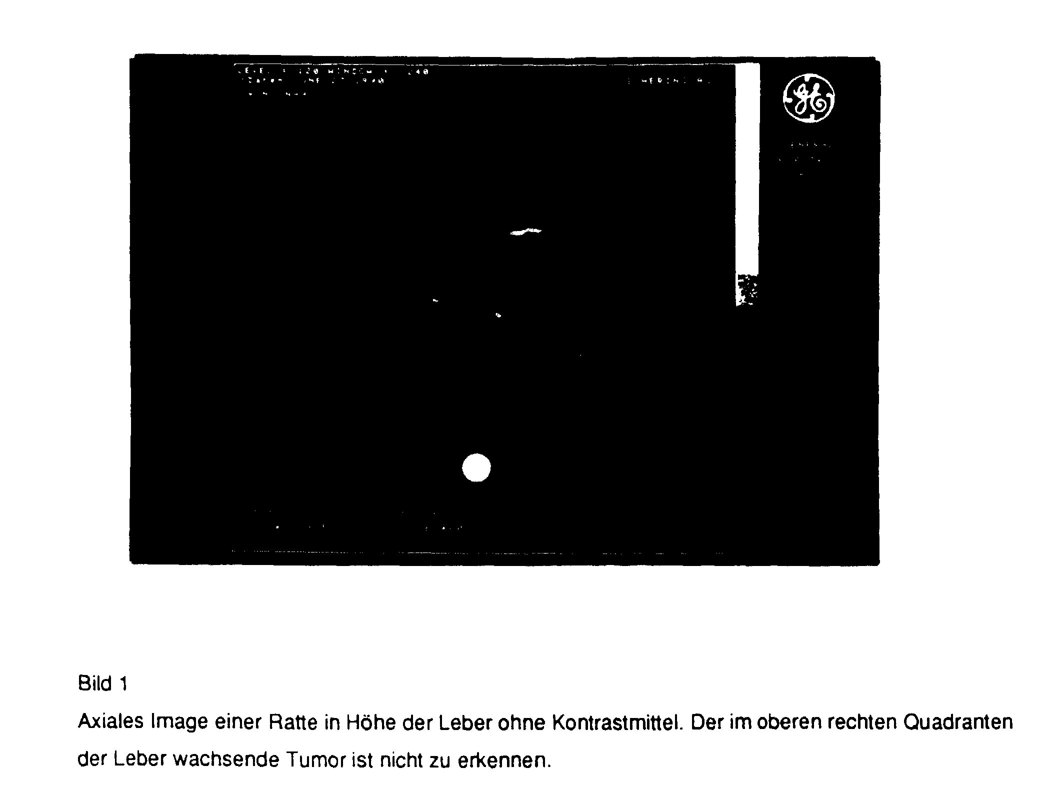

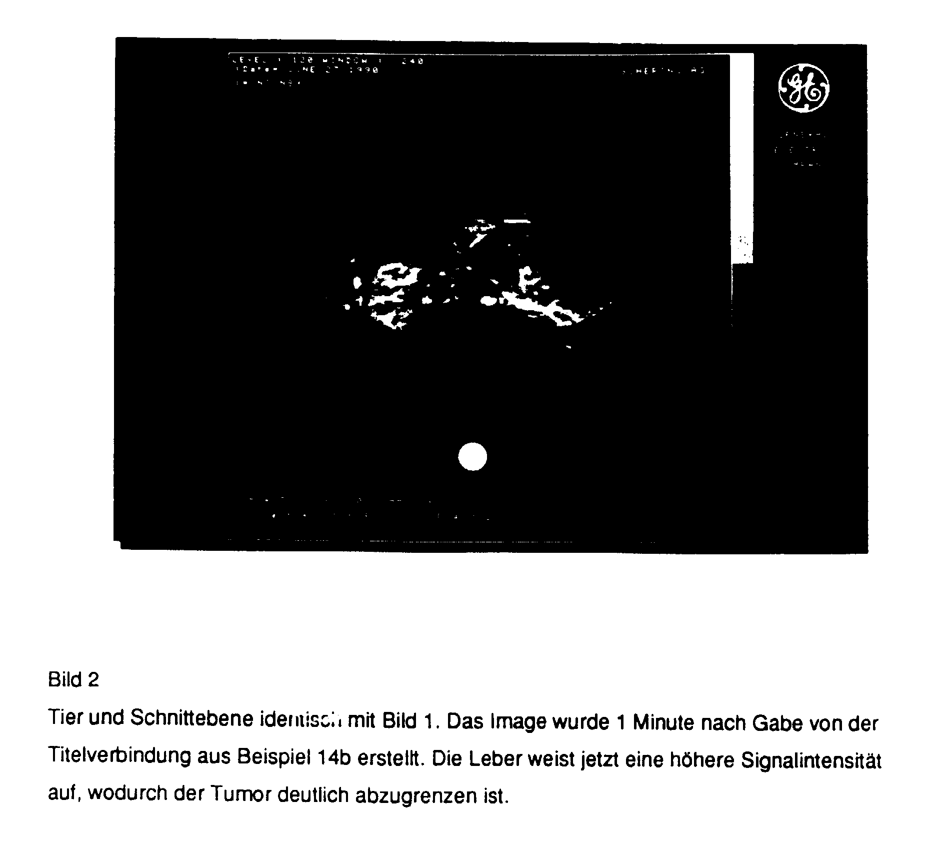

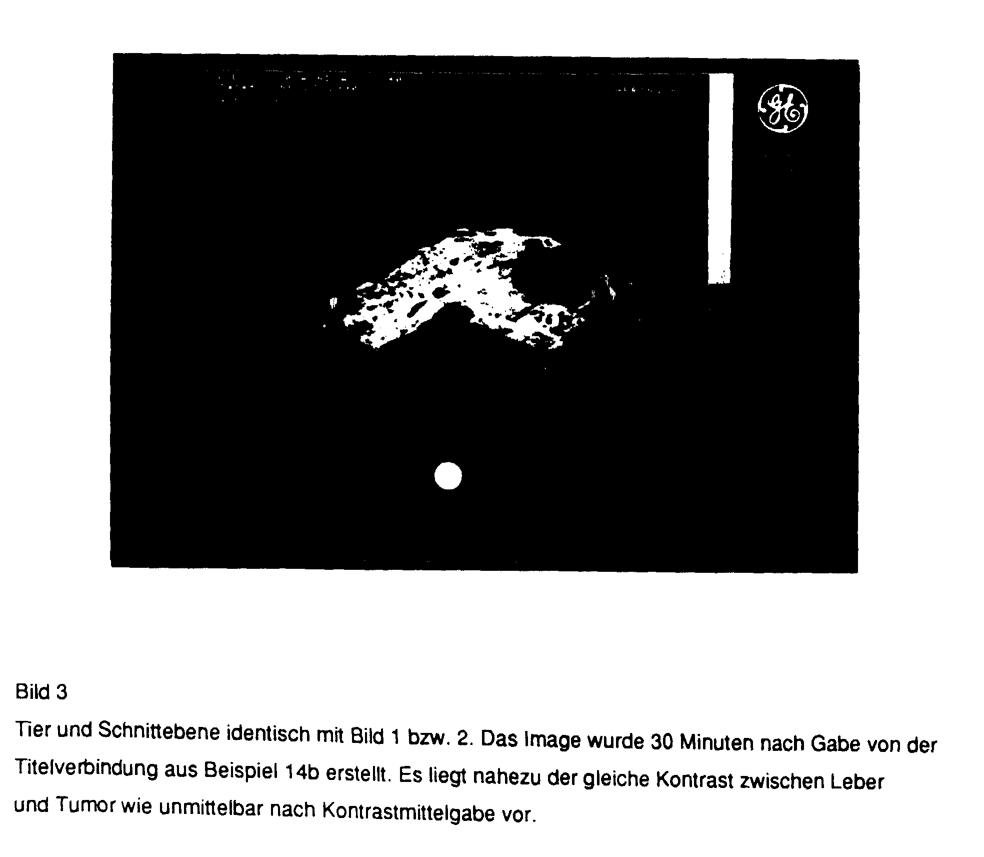

## Patentansprüche

1. Tetraazacyclododecan-Verbindungen der allgemeinen Formel I 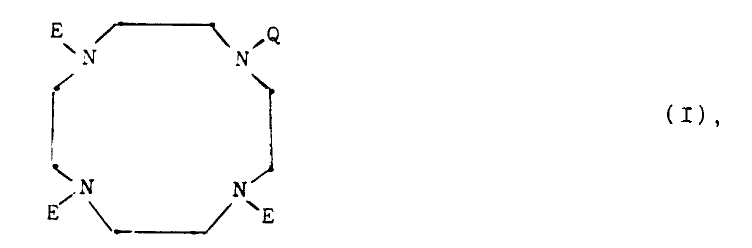
E für einen Rest -CH(R¹)-CO₂Y mit Y in der Bedeutung eines Wasserstoffatoms und/oder eines Metallionenäquivalents eines Elements der Ordnungszahlen 21-29, 31, 32, 37-39, 42-44, 47, 49 oder 57-83 und R¹ in der Bedeutung eines Wasserstoffatoms, einer gegebenenfalls mit 1 bis 6 Hydroxygruppen substituierten verzweigten oder unverzweigten C₁-C₃₀-Alkyl-, C₆-C₁₀-Aryl- oder C₇-C₃₀-Aralkylgruppe,
Q für einen Rest -CH(R')-CH(OH)R
mit R in der Bedeutung eines 1 bis 10 Sauerstoffatome enthaltenden und gegebenenfalls durch 1 bis 6 Hydroxy- oder OCOR²-Gruppen mit R² in der Bedeutung eines Wasserstoffatoms oder eines verzweigten oder unverzweigten C₁-C₃₀-Alkyl-, C₆-C₁₀-Aryl- oder C₇-C₃₀-Aralkylrestes
substituierten verzweigten oder unverzweigten C₁-C₃₀-Alkyl- oder C₇-C₃₀-Aralkylrestes,
der gegebenenfalls 1 bis 2 -OCO-Gruppen, 1 bis 2 Cyclohexan- und/oder Cyclohexylenreste (die ihrerseits durch 1 bis 3 (CH₂)ₖCOOR²-Gruppen mit k in der Bedeutung der Ziffern 0 bis 10 substituiert sein können), gegebenenfalls 1 bis 5 C₁-C₇-Alkoxy-, C₆-C₁₀-Aryloxy- oder C₇-C₁₀-Aralkoxygruppen, gegebenenfalls einen Rest -NR²-COR³ oder -CONR²R³, wobei R³ die für R² angegebene Bedeutung hat, und/oder 1 bis 2 CO₂R²-Reste enthalt und deren gegebenenfalls in der Kette enthaltenen Arylreste durch 1 bis 3, gegebenenfalls 1 bis 5 Sauerstoffatome, 1 bis 3 Hydroxyreste, 1 bis 5 C₁-C₇-Alkoxygruppen und/oder 1 bis 3 (CH₂)ₖCOOR²-Gruppen enthaltende verzweigte oder unverzweigte C₁-C₃₀-Alkyl-, C₆-C₁₀-Aryl- oder C₇-C₃₀-Aralkylreste, 1 bis 3 F-, Cl-, Br-, OH-, NO₂-, NH₂-, NCS-, CO₂R², NHCOCH₂Cl-, NHCOCH₂Br-, NHCOCH₂R²-, CON₃-Reste substituiert sein können, und
R' in der Bedeutung eines Wasserstoffatoms oder von R, wobei, wenn R' für ein Wasserstoffatom steht, im Falle einer 2-Oxa-C₁-C₃₀-Alkylkette diese - zusätzlich zu einer gegebenenfalls vorhandenen endständigen Methoxy- oder Ethoxygruppe - durch mindestens eine der oben angegebenen Reste substituiert sein muß,
oder für ein über eine Bis(β-hydroxy)-alkylenkette 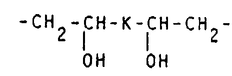 gebundenes zweites Tetraazacyclododecan-Molekül mit K in der Bedeutung einer 1 bis 6 Sauerstoffatome, gegebenenfalls 1 bis 2 Benzyloxy-, Phenylen-, Phenylenoxy- und/oder Phenylendioxygruppen enthaltenen, gegebenenfalls durch 1 bis 6 Hydroxy-, 1 bis 6 Hydroxy-C₁-C₆-alkyl- und/oder 1 bis 8 C₁-C₇-Alkoxy- oder Aralkoxygruppen substituierten C₁-C₁₆-Alkylenkette
stehen sowie deren Salze mit anorganischen und/oder organischen Basen, Aminosäuren oder Aminosaureamiden.

2. Verbindungen gemäß Anspruch 1, dadurch gekennzeichnet, daß Y für Wasserstoffatome steht.

3. Verbindungen gemäß Anspruch 1, dadurch gekennzeichnet, daß mindestens zwei der Substituenten Y Metallionenäquivalente mindestens eines Elements der Ordnungszahlen 21-29, 42, 44 oder 57-83 oder mindestens eines Radionuklids eines Elements der Ordnungszahlen 21, 26, 27, 29, 31, 32, 37-39, 43, 47, 49, 62-64, 67, 70, 71, 75, 77, 79 und 83 sind.

4. Verbindungen gemäß Anspruch 1, dadurch gekennzeichnet, daß R¹ für ein Wasserstoffatom steht.

5. Verbindungen gemäß Anspruch 1, dadurch gekennzeichnet, daß R' für ein Wasserstoffatom steht.

6. Pharmazeutische Mittel enthaltend mindestens eine physiologisch verträgliche Verbindung nach Anspruch 1, gegebenenfalls mit den in der Galenik üblichen Zusätzen.

7. Verwendung von mindestens einer physiologisch verträglichen Verbindung gemäß Anspruch 1 für die Herstellung von Mitteln für die NMR-, Röntgen-, Radio-Diagnostik, Radio- oder Strahlentherapie.

8. Verfahren zur Herstellung von Tetraazacyclododecan-Verbindungen der allgemeinen Formel I 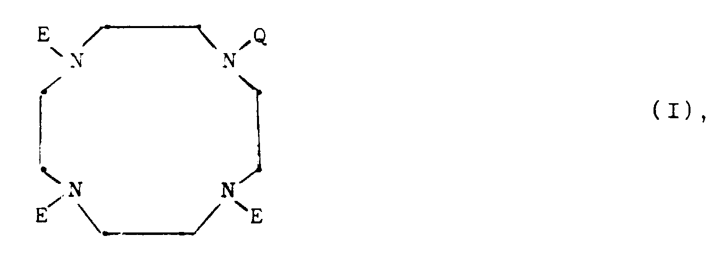 worin
E für einen Rest -CH(R¹)-CO₂Y mit Y in der Bedeutung eines Wasserstoffatoms und/oder eines Metallionenäquivalents eines Elements der Ordnungszahlen 21-29, 31, 32, 37-39, 42-44, 47, 49 oder 57-83 und R¹ in der Bedeutung eines Wasserstoffatoms, einer gegebenenfalls mit 1 bis 6 Hydroxygruppen substituierten verzweigten oder unverzweigten C₁-C₃₀-Alkyl-, C₆-C₁₀-Aryl- oder C₇-C₃₀-Aralkylgruppe,
Q für einen Rest -CH(R')-CH(OH)R
mit R in der Bedeutung eines 1 bis 10 Sauerstoffatome enthaltenden und gegebenenfalls durch 1 bis 6 Hydroxy- oder OCOR²-Gruppen mit R² in der Bedeutung eines Wasserstoffatoms oder eines verzweigten oder unverzweigten C₁-C₃₀-Alkyl-, C₆-C₁₀-Aryl- oder C₇-C₃₀-Aralkylrestes
substituierten verzweigten oder unverzweigten C₁-C₃₀-Alkyl- oder C₇-C₃₀-Aralkylrestes,
der gegebenenfalls 1 bis 2 -OCO-Gruppen, 1 bis 2 Cyclohexan- und/oder Cyclohexylenreste (die ihrerseits durch 1 bis 3 (CH₂)ₖCOOR²-Gruppen mit k in der Bedeutung der Ziffern 0 bis 10 substituiert sein können), gegebenenfalls 1 bis 5 C₁-C₇-Alkoxy-, C₆-C₁₀-Aryloxy- oder C₇-C₁₀-Aralkoxygruppen, gegebenenfalls einen Rest -NR²-COR³ oder -CONR²R³, wobei R³ die für R² angegebene Bedeutung hat, und/oder 1 bis 2 CO₂R²-Reste enthält und deren gegebenenfalls in der Kette enthaltenen Arylreste durch 1 bis 3, gegebenenfalls 1 bis 5 Sauerstoffatome, 1 bis 3 Hydroxyreste, 1 bis 5 C₁-C₇-Alkoxygruppen und/oder 1 bis 3 (CH₂)ₖCOOR²-Gruppen enthaltende verzweigte oder unverzweigte C₁-C₃₀-Alkyl-, C₆-C₁₀-Aryl- oder C₇-C₃₀-Aralkylreste, 1 bis 3 F-, Cl-, Br-, OH-, NO₂-, NH₂,- NCS-, CO₂R², NHCOCH₂Cl-, NHCOCH₂Br-, NHCOCH₂R²-, CON₃-Reste substituiert sein können, und
R' in der Bedeutung eines Wasserstoffatoms oder von R, wobei, wenn R' für ein Wasserstoffatom steht, im Falle einer 2-Oxa-C₁-C₃₀-Alkylkette diese - zusätzlich zu einer gegebenenfalls vorhandenen endständigen Methoxy- oder Ethoxygruppe - durch mindestens eine der oben angegebenen Reste substituiert sein muß,
oder für ein über eine Bis(β-hydroxy)-alkylenkette 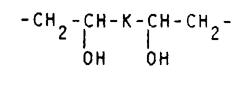 gebundenes zweites Tetraazacyclododecan-Molekül mit K in der Bedeutung einer 1 bis 6 Sauerstoffatome, gegebenenfalls 1 bis 2 Benzyloxy-, Phenylen-, Phenylenoxy- und/oder Phenylendioxygruppen enthaltenen, gegebenenfalls durch 1 bis 6 Hydroxy-, 1 bis 6 Hydroxy-C₁-C₆-alkyl- und/oder 1 bis 8 C₁-C₇-Alkoxy- oder Aralkoxygruppen substituierten C₁-C₁₆-Alkylenkette
stehen sowie deren Salze mit anorganischen und/oder organischen Basen, Aminosäuren oder Aminosäureamiden,
dadurch gekennzeichnet, daß man Verbindungen der allgemeinen Formel II 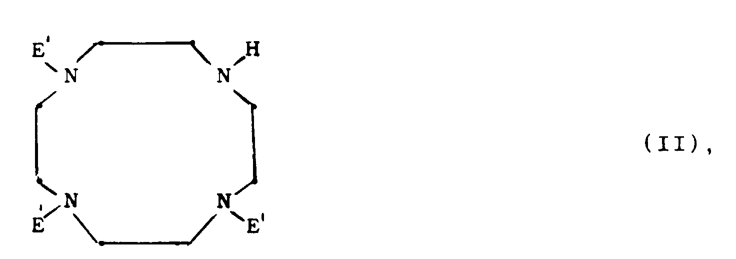 worin
E' den für E angegebenen Rest, in dem darin gegebenenfalls vorhandene Hydroxygruppen gegebenenfalls geschützt sind,
bedeutet,
mit einem Edukt der allgemeinen Formel III bzw. IV 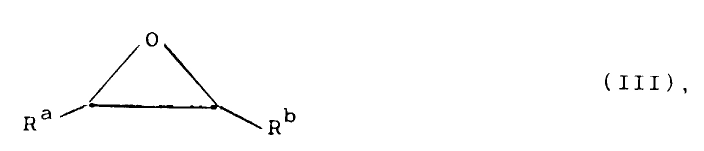 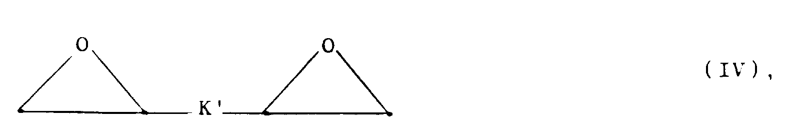 worin
K' den fur K angegebenen Rest, in dem darin gegebenenfalls vorhandene Hydroxygruppen gegebenenfalls geschützt sind, bedeutet
R^{a} und R^{b} die für R' und R angegebene Bedeutung, jedoch keine Gruppen NH₂, NCS, NHCOCH₂Cl oder NHCOCH₂Br enthalten und gegebenenfalls vorhandene Carboxylgruppen gewünschtenfalls in geschützter Form vorliegen, haben,
in Wasser oder in mit Wasser mischbaren Lösungsmitteln wie z.B. Acetonitril, DMF, DMA, Ethanol, Methanol, Dioxan, THF, DMSO, DME oder deren Gemischen bei Temperaturen von 0 °C bis 170 °C, vorzugsweise 20 bis 100 °C, innerhalb von 12 bis 48 Stunden, vorzugsweise 12 bis 24 Stunden, unter Zusatz von anorganischen und/oder organischen Basen, wie z.B. Kalium-, Natrium-, Lithium-, Calcium-, Barium-, Magnesiumhydroxid, Natrium-, Kaliumcarbonat, Triethyl-, Tripropyl-, Tributylamin, Pyridin, DMAP, Reillex^{(R)}, Triton B^{(R)} umsetzt, gegebenenfalls noch vorhandene Schutzgruppen spaltet, die so erhaltenen Komplexbildner (Y steht für Wasserstoffatome) mit mindestens einem Metalloxid oder Metallsalz eines Elements der Ordnungszahlen 21-29, 31, 32, 37-39, 42-44, 47, 49 oder 57-83 umsetzt und anschließend - falls gewünscht - vorhandene acide Wasserstoffatome durch Kationen anorganischer und/oder organischer Basen, Aminosäuren oder Aminosäureamide substituiert, wobei - falls die gewünschten Endprodukte die Gruppen NH₂, NCS, NHCOCH₂Cl, NHCOCH₂Br bzw. CON₃ enthalten sollen - man von Edukten der allgemeinen Formel III ausgeht, die anstelle dieser Gruppen NO₂- bzw. CO₂R²-Gruppen enthalten, anschließend die letztgenannten Gruppen in die oben genannten gewünschten funktionellen Gruppen umwandelt, wobei diese Umwandlung vor oder nach der Komplexierung der Komplexbildner und anschließender Substitution gegebenenfalls noch vorhandener acider Wasserstoffatome mit den jeweils gewünschten Metallionen erfolgen kann.

9. Verfahren zur Herstellung der pharmazeutischen Mittel gemäß Anspruch 6, dadurch gekennzeichnet, daß man die in Wasser, physiologischer Salz- oder Proteinlösung gelöste oder suspendierte Komplexverbindung, gegebenenfalls mit den in der Galenik üblichen Zusätzen, in eine für die enterale oder parenterale Applikation geeignete Form bringt.

## Claims

1. Tetraazacyclododecane compounds of the general formula I 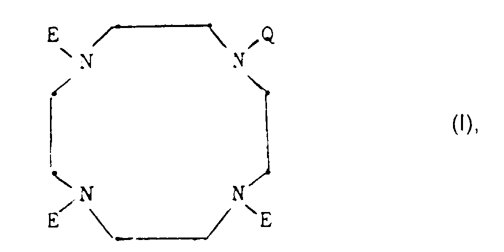 wherein
E represents a radical -CH(R¹)-CO₂Y, wherein Y denotes a hydrogen atom and/or a metal ion equivalent of an element having an atomic number of from 21 to 29, 31, 32, from 37 to 39, from 42 to 44, 47, 49 or from 57 to 83 and R¹ denotes a hydrogen atom or a branched or unbranched C₁-C₃₀alkyl, C₆-C₁₀aryl or C₇-C₃₀aralkyl group each optionally substituted by from 1 to 6 hydroxy groups,
Q represents a radical -CH(R')-CH(OH)R,
wherein R denotes a branched or unbranched C₁-C₃₀alkyl or C₇-C₃₀aralkyl radical that contains from 1 to 10 oxygen atoms and is optionally substituted by from 1 to 6 hydroxy or OCOR² groups,
R² denoting a hydrogen atom or a branched or unbranched C₁-C₃₀alkyl, C₆-C₁₀aryl or C₇-C₃₀aralkyl radical,
and that contains optionally 1 or 2 -OCO- groups, 1 or 2 cyclohexane and/or cyclohexylene radicals (which may in turn be substituted by from 1 to 3 (CH₂)ₖCOOR² groups wherein k denotes a number from 0 to 10), optionally from 1 to 5 C₁-C₇alkoxy, C₆-C₁₀aryloxy or C₇-C₁₀aralkoxy groups, optionally a radical -NR²-COR³ or -CONR²R³, wherein R³ is as defined for R², and/or 1 or 2 CO₂R² radicals, and the aryl radicals thereof optionally present in the chain may be substituted by from 1 to 3 branched or unbranched C₁-C₃₀alkyl, C₆-C₁₀aryl or C₇-C₃₀aralkyl radicals (each of which may contain from 1 to 5 oxygen atoms, from 1 to 3 hydroxy radicals, from 1 to 5 C₁-C₇alkoxy groups and/or from 1 to 3 (CH₂)ₖCOOR² groups), and/or by from 1 to 3 F, Cl, Br, OH, NO₂, NH₂, NCS, CO₂R², NHCOCH₂Cl, NHCOCH₂Br, NHCOOH₂R² or CON₃ radicals,
and R' denotes a hydrogen atom or has the same meaning as R,
with the proviso that, when R' is a hydrogen atom, in the case of a 2-oxa-C₁-C₃₀alkyl chain, that chain must be substituted by at least one of the radicals mentioned above in addition to any terminal methoxy or ethoxy group which may be present,
or Q represents a second tetraazacyclododecane molecule bound by means of a bis-(β-hydroxy)-alkylene chain 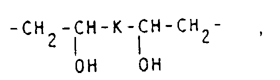 wherein K denotes a C₁-C₁₆alkylene chain that contains from 1 to 6 oxygen atoms and optionally 1 or 2 benzyloxy, phenylene, phenyleneoxy and/or phenylenedioxy groups and is optionally substituted by from 1 to 6 hydroxy, from 1 to 6 hydroxy-C₁-C₆alkyl and/or from 1 to 8 C₁-C₇alkoxy or aralkoxy groups,
and salts thereof with inorganic and/or organic bases, amino acids or amino acid amides.

2. Compounds according to claim 1, characterised in that each Y represents a hydrogen atom.

3. Compounds according to claim 1, characterised in that at least two of the substituents Y are metal ion equivalents of at least one element having an atomic number of from 21 to 29, 42, 44 or from 57 to 83 or of at least one radionuclide of an element having an atomic number of 21, 26, 27, 29, 31, 32, from 37 to 39, 43, 47, 49, from 62 to 64, 67, 70, 71, 75, 77, 79 or 83.

4. Compounds according to claim 1, characterised in that R¹ represents a hydrogen atom.

5. Compounds according to claim 1, characterised in that R' represents a hydrogen atom.

6. Pharmaceutical compositions comprising at least one physiologically tolerable compound according to claim 1, optionally together with the additives customary in galenical pharmacy.

7. Use of at least one physiologically tolerable compound according to claim 1 for the preparation of compositions for NMR diagnostics, X-ray diagnostics, radiodiagnostics, radiotherapy or radiation therapy.

8. Process for the preparation of tetraazacyclododecane compounds of the general formula I 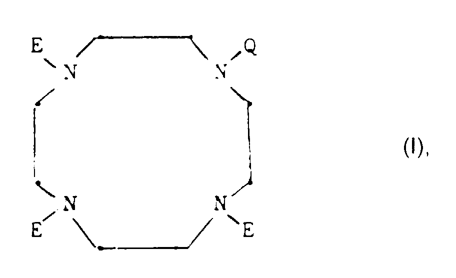 wherein
E represents a radical -CH(R¹)-CO₂Y, wherein Y denotes a hydrogen atom and/or a metal ion equivalent of an element having an atomic number of from 21 to 29, 31, 32, from 37 to 39, from 42 to 44, 47, 49 or from 57 to 83 and R¹ denotes a hydrogen atom or a branched or unbranched C₁-C₃₀alkyl, C₆-C₁₀aryl or C₇-C₃₀aralkyl group each optionally substituted by from 1 to 6 hydroxy groups,
Q represents a radical -CH(R')-CH(OH)R,
wherein R denotes a branched or unbranched C₁-C₃₀alkyl or C₇-C₃₀aralkyl radical that contains from 1 to 10 oxygen atoms and is optionally substituted by from 1 to 6 hydroxy or OCOR² groups,
R² denoting a hydrogen atom or a branched or unbranched C₁-C₃₀alkyl, C₆-C₁₀aryl or C₇-C₃₀aralkyl radical,
and that contains optionally 1 or 2 -OCO- groups, 1 or 2 cyclohexane and/or cyclohexylene radicals (which may in turn be substituted by from 1 to 3 (CH₂)ₖCOOR² groups wherein k denotes a number from 0 to 10), optionally from 1 to 5 C₁-C₇alkoxy, C₆-C₁₀aryloxy or C₇-C₁₀aralkoxy groups, optionally a radical -NR²-COR³ or -CONR²R³, wherein R³ is as defined for R², and/or 1 or 2 CO₂R² radicals, and the aryl radicals thereof optionally present in the chain may be substituted by from 1 to 3 branched or unbranched C₁-C₃₀alkyl, C₆-C₁₀aryl or C₇-C₃₀aralkyl radicals (each of which may contain from 1 to 5 oxygen atoms, from 1 to 3 hydroxy radicals, from 1 to 5 C₁-C₇alkoxy groups and/or from 1 to 3 (CH₂)ₖCOOR² groups), and/or by from 1 to 3 F, Cl, Br, OH, NO₂, NH₂, NCS, CO₂R², NHCOCH₂Cl, NHCOCH₂Br, NHCOCH₂R² or CON₃ radicals,
and R' denotes a hydrogen atom or has the same meaning as R, with the proviso that, when R' is a hydrogen atom, in the case of a 2-oxa-C₁-C₃₀alkyl chain, that chain must be substituted by at least one of the radicals mentioned above in addition to any terminal methoxy or ethoxy group which may be present,
or Q represents a second tetraazacyclododecane molecule bound by means of a bis-(β-hydroxy)-alkylene chain 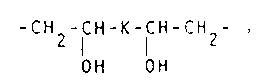 wherein K denotes a C₁-C₁₆alkylene chain that contains from 1 to 6 oxygen atoms and optionally 1 or 2 benzyloxy, phenylene, phenyleneoxy and/or phenylenedioxy groups and is optionally substituted by from 1 to 6 hydroxy, from 1 to 6 hydroxy-C₁-C₆alkyl and/or from 1 to 8 C₁-C₇alkoxy or aralkoxy groups,
and salts thereof with inorganic and/or organic bases, amino acids or amino acid amides, characterised in that compounds of the general formula II 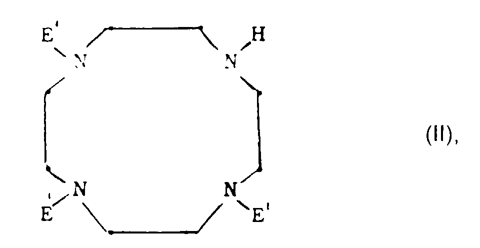 wherein
E' represents the radical defined for E, in which any hydroxy groups present are optionally protected,
are reacted with a starting product of the general formula III or IV 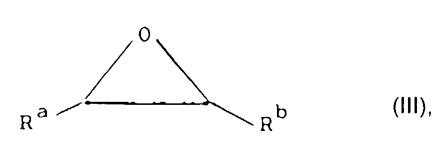 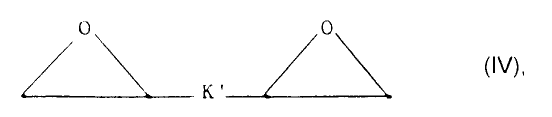 wherein
K' represents the radical defined for K, in which any hydroxy groups present are optionally protected,
R^{a} and R^{b} are as defined for R' and R but contain no NH₂, NCS, NHCOCH₂Cl or NHCOCH₂Br groups and any carboxy groups present are optionally in protected form,
in water or in water-miscible solvents, such as, for example, acetonitrile, DMF, DMA, ethanol, methanol, dioxane, THF, DMSO and DME or mixtures thereof, at temperatures of from 0°C to 170°C, preferably from 20 to 100°C, within a period of from 12 to 48 hours, preferably from 12 to 24 hours, with addition of inorganic and/or organic bases, such as, for example, potassium, sodium, lithium, calcium, barium or magnesium hydroxide, sodium or potassium carbonate, triethyl-, tripropyl- or tributyl-amine, pyridine, DMAP, Reillex® and Triton B®, removing any protecting groups that may still be present, reacting the complexing agents thereby obtained (Y represents hydrogen atoms) with at least one metal oxide or metal salt of an element having an atomic number of from 21 to 29, 31, 32, from 37 to 39, from 42 to 44, 47, 49 or from 57 to 83 and then - if desired - replacing any acid hydrogen atoms present by cations of inorganic and/or organic bases, amino acids or amino acid amides,
and, if the desired end products are to contain the groups NH₂, NCS, NHCOCH₂Cl, NHCOCH₂Br and/or CON₃, starting products of the general formula III are used that contain, instead of those groups, NO₂ or CO₂R² groups and the last-mentioned groups are then converted into the desired functional groups mentioned above, it being possible for that conversion to be performed before or after the complexing of the complexing agents and the subsequent replacement of acid hydrogen atoms that may still be present by the metal ions desired in the particular case.

9. Process for the preparation of the pharmaceutical compositions according to claim 6, characterised in that the complex compound, dissolved or suspended in water, physiological saline solution or protein solution, optionally together with the additives customary in galenical pharmacy, is brought into a form suitable for enteral or parenteral administration.

## Revendications

1. Composés de tétraazacyclododécane de la formule générale I: 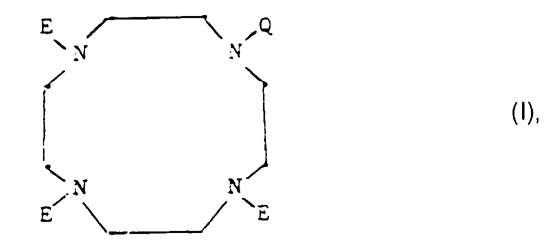 dans laquelle E représente un radical -CH(R¹)- CO₂Y, où Y a la signification d'un atome d'hydrogène et/ou d'un équivalent d'ion métallique d'un élément des nombres atomiques 21-29, 31, 32, 37-39, 42-44, 47, 49 ou 57-83 et R¹ a la signification d'un atome d'hydrogène, ou d'un groupe aralkyle en C₇-C₃₀, aryle en C₆-C₁₀, ou alkyle en C₁-C₃₀, ramifié ou non ramifié, éventuellement substitué par 1 à 6 groupes hydroxy,
Q représente un radical -CH(R')-CH(OH)R,
où R représente un radical aralkyle en C₇-C₃₀ ou alkyle en C₁-C₃₀, ramifié ou non ramifié, qui contient 1 à 10 atomes d'oxygène et est éventuellement substitué par 1 à 6 groupes hydroxy ou OCOR², où R² a la signification d'un atome d'hydrogène ou d'un radical aralkyle en C₇-C₃₀, aryle en C₆-C₁₀ ou alkyle en C₁-C₃₀, ramifié ou non ramifié, et qui contient éventuellement 1 à 2 groupes -OCO-, 1 à 2 radicaux cyclohexane et/ou cyclohexylène (qui à leur tour peuvent être substitués par 1 à 3 groupes (CH₂)ₖCOOR², où k peut avoir la signification des chiffres 0 à 10), éventuellement 1 à 5 groupes alcoxy en C₁-C₇, aryloxy en C₆-C₁₀ ou aralcoxy en C₇-C₁₀, éventuellement 1 radical -NR²-COR³ ou -CONR²R³, où R³ a la signification donnée pour R², et/ou 1 à 2 radicaux CO₂R² et dont les radicaux aryle éventuellement contenus dans la chaîne, peuvent être substitués par 1 à 3, éventuellement 1 à 5, atomes d'oxygène, 1 à 3 radicaux hydroxyle, des radicaux aralkyle en C₇-C₃₀, aryle en C₆-C₁₀ ou alkyle en C₁-C₃₀, ramifiés ou non ramifiés, contenant 1 à 5 groupes alcoxy en C₁-C₇ et/ou 1 à 3 groupes (CH₂)ₖCOOR², 1 à 3 radicaux F-, Cl-, Br-, OH-, NO₂-, NH₂-, NCS-, CO₂R², NHCOCH₂Cl-, NHCOCH₂Br-, NHCOCH₂R²-, CON₃-, et
R' représente un atome d'hydrogène ou R, étant entendu que, lorsque R' représente un atome d'hydrogène, dans le cas d'une chaîne 2-oxa-alkyle en C₁-C₃₀, celle-ci doit être substituée, en supplément à un groupe méthoxy ou éthoxy de fin de chaîne éventuellement présent, par au moins un des radicaux indiqués ci-dessus,
ou une deuxième molécule de tétraazacyclododécane fixée par l'intermédiaire d'une chaîne bis-(β-hydroxy)-alkylène 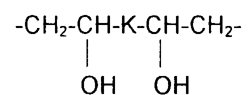 où K a la signification d'une chaîne alkylène en C₁-C₁₆ contenant 1 à 6 atomes d'oxygène, éventuellement 1 à 2 groupes benzyloxy, phénylène, phénylèneoxy et/ou phénylènedioxy, et éventuellement substituée par 1 à 6 groupes hydroxy, 1 à 6 groupes hydroxy-alkyle en C₁-C₆ et/ou 1 à 8 groupes aralcoxy ou alcoxy en C₁-C₇,
ainsi que leurs sels avec des bases inorganiques et/ou organiques, des acides aminés ou des amides d'acides aminés.

2. Composés suivant la revendication 1, caractérisés en ce que Y représente des atomes d'hydrogène.

3. Composés suivant la revendication 1, caractérisés en ce qu'au moins deux des substituants Y sont des équivalents d'ions métalliques d'au moins un élément des nombres atomiques 21-29, 42, 44 ou 57-83 ou au moins un radionuclide d'un élément des nombres atomiques 21, 26, 27, 29, 31, 32, 37-39, 43, 47, 49, 62-64, 67, 70, 71, 75, 77, 79 et 83.

4. Composés suivant la revendication 1, caractérisés en ce que R¹ représente un atome d'hydrogène.

5. Composés suivant la revendication 1, caractérisés en ce que R' représente un atome d'hydrogène.

6. Produits pharmaceutiques contenant au moins un composé physiologiquement compatible suivant la revendication 1, éventuellement avec les additifs courants en pharmacie galénique.

7. Utilisation d'au moins un composé physiologiquement compatible suivant la revendication 1 pour la préparation de produits pour le diagnostic à RMN, aux rayons X, le radiodiagnostic, la radiothérapie ou la thérapie par les rayons.

8. Procédé de préparation de composés de tétraazacyclododécane de la formule générale I 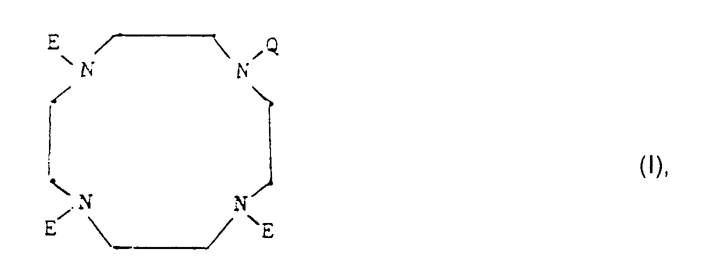 dans laquelle
E représente un radical -CH(R¹)-CO₂Y où Y représente un atome d'hydrogène et/ou un équivalent d'ion métallique d'un élément des nombres atomiques 21-29, 31, 32, 37-39, 42-44, 47, 49 ou 57-83, et R¹ représente un atome d'hydrogène ou un groupe aralkyle en C₇-C₃₀, aryle en C₆-C₁₀ ou alkyle en C₁-C₃₀, ramifié ou non ramifié, éventuellement substitué par 1 à 6 groupes hydroxy,
Q représente un radical -CH(R')-CH(OH)R
où R a la signification d'un radical aralkyle en C₇-C₃₀ ou alkyle en C₁-C₃₀, ramifié ou non ramifié, qui contient 1 à 10 atomes d'oxygène et est éventuellement substitué par 1 à 6 groupes hydroxy ou OCOR², où R² a la signification d'un atome d'hydrogène ou d'un radical aralkyle en C₇-C₃₀, aryle en C₆-C₁₀ ou alkyle en C₁-C₃₀, ramifié ou non ramifié, et qui contient éventuellement 1 à 2 groupes -OCO-, 1 à 2 radicaux cyclohexane et/ou cyclohexylène (qui à leur tour peuvent être substitués par 1 à 3 groupes (CH₂)ₖCOOR², où k a la signification des chiffres 0 à 10), éventuellement 1 à 5 alcoxy en C₁-C₇, aryloxy en C₆-C₁₀ ou aralcoxy en C₇-C₁₀, éventuellement un radical -NR²-COR³ ou -CONR²R³, où R³ a la signification donnée pour R², et/ou 1 à 2 radicaux CO₂R², et dont les radicaux aryle éventuellement contenus dans la chaîne peuvent être substitués par 1 à 3, éventuellement 1 à 5, atomes d'oxygène, 1 à 3 radicaux hydroxy, des radicaux aralkyle en C₇-C₃₀, aryle en C₆-C₁₀ et alkyle en C₁-C₃₀, ramifiés ou non ramifiés qui contiennent 1 à 5 groupes alcoxy en C₁-C₇ et/ou 1 à 3 groupes (CH₂)ₖ COOR², 1 à 3 radicaux F-, Cl-, Br-, OH-, NO₂-, NH₂-, NCS-, CO₂R², NHCOCH₂Cl-, NHCOCH₂Br-, NHCOCH₂R²-, CON₃-, et
R' représente un atome d'hydrogène ou R, étant entendu que, lorsque R' représente un atome d'hydrogène, dans le cas d'une chaîne 2-oxa-alkyle en C₁-C₃₀, celle-ci doit être substituée, en supplément à un groupe méthoxy ou éthoxy en fin de chaîne, éventuellement présent, par au moins un des radicaux indiqués ci-dessus,
ou une deuxième molécule de trétraazacyclododécane liée par l'intermédiaire d'une chaîne bis(β-hydroxy)-alkylène 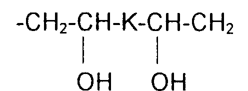
où K a la signification d'une chaîne alkylène en C₁-C₆ contenant 1 à 6 atomes d'oxygène, éventuellement 1 à 2 groupes benzyloxy, phénylène, phénylèneoxy et/ou phénylènedioxy, et éventuellement substituée par 1 à 6 groupes hydroxy, 1 à 6 groupes hydroxy-alkyle en C₁-C₆ et/ou 1 à 8 groupes aralcoxy ou alcoxy en C₁-C₇,
ainsi que de leurs sels avec des bases inorganiques et/ou organiques, des acides aminés ou des amides d'acides aminés,
caractérisé en ce qu'on fait réagir des composés de la formule générale II 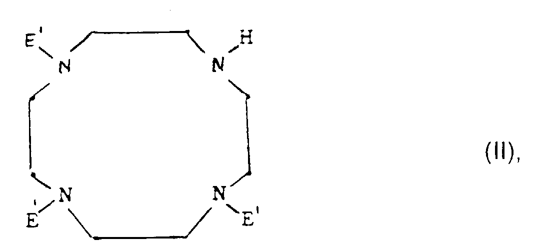 dans laquelle
E' représente le radical indiqué pour E, dans lequel des groupes hydroxy éventuellement présents sont éventuellement protégés,
avec un produit d'émission de la formule générale III ou IV 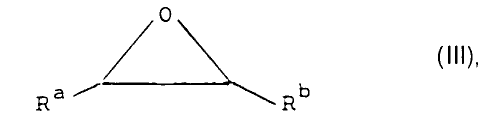 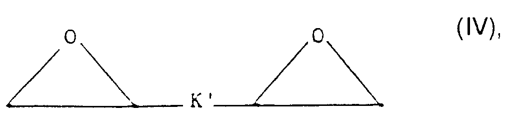 dans lesquelles
K' représente le radical indiqué pour K, dans lequel des groupes hydroxy éventuellement présents sont éventuellement protégés,
R^{a} et R^{b} ont la signification indiquée pour R' et R, mais ils ne contiennent aucun groupe NH₂, NCS, NHCOCH₂Cl ou NHCOCH₂Br et présentent des groupes carboxyle éventuellement présents, éventuellement sous forme protégée,
dans de l'eau ou dans des solvants miscibles à l'eau, comme par exemple l'acétonitrile, du DMF, du DMA, de l'éthanol, du méthanol, du dioxane, du THF, du DMSO, du DME ou leurs mélanges, à des températures de 0 à 170°C, de préférence de 20 à 100°C, en l'espace de 12 à 48 heures, de préférence de 12 à 24 heures, avec addition de bases inorganiques et/ou organiques, comme par exemple de l'hydroxyde de potassium, de sodium, de lithium, de calcium, de baryum, de magnésium, du carbonate de sodium, de potassium, de la triéthylamine, de la tripropylamine, de la tributylamine, de la pyridine, de la DMAP, du Reillex®, Triton B®, on sépare éventuellement des groupes de protection encore présents, on fait réagir le complexant ainsi obtenu (Y représente des atomes d'hydrogène) avec au moins un oxyde métallique ou un sel métallique d'un élément des nombres atomiques 21-29, 31, 32, 37-39, 42-44, 47, 49 ou 57-83, et ensuite, en cas de besoin, on substitue des atomes d'hydrogène acides présents par des cations de bases inorganiques et/ou organiques, d'acides aminés ou d'amides d'acides aminés, tandis que, dans le cas où les produits finals souhaités doivent contenir les groupes NH₂, NCS, NHCOCH₂Cl, NHCOCH₂Br ou CON₃, on part de produits d'émission de la formule générale III, qui, au lieu de ces groupes, contiennent des groupes NO₂- ou CO₂R², puis on transforme les groupes cités en dernier lieu en les groupes fonctionnels souhaités cités ci-dessus, cette transformation pouvant avoir lieu avant ou après la complexation des complexants, et une substitution ultérieure d'atomes d'hydrogène acides éventuellement encore présents par les ions métalliques respectivement souhaités.

9. Procédé de préparation des produits pharmaceutiques selon la revendication 6, caractérisé en ce qu'on amène sous une forme appropriée pour l'application entérale ou parentérale le composé complexe en solution ou en suspension dans l'eau, dans une solution saline physiologique ou dans une solution protéique, éventuellement avec les additifs courants en pharmacie galénique.
